(19) Europäisches Patentamt
European Patent Office
Office européen des brevets

(11) Publication number: **0 314 239 A2**

# (12) EUROPEAN PATENT APPLICATION

(21) Application number: 88202334.4

(22) Date of filing: 19.10.88

(51) Int. Cl.4: C07K 5/02 , C07K 5/06 , A61K 37/64

(30) Priority: 28.10.87 US 113681

(43) Date of publication of application: 03.05.89 Bulletin 89/18

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Greenlee, William J.
115 Herrick Avenue
Teaneck New Jersey 07666(US)
Inventor: Parsons, William H.
2171 Oliver Street
Rahway New Jersey 07065(US)

(74) Representative: Hesketh, Alan, Dr.
European Patent Department Merck & Co., Inc. Terlings Park Eastwick Road
Harlow Essex, CM20 2QR(GB)

## (54) Tripeptide renin inhibitors with N-terminal ureido or sulfamido groups.

(57) Tripeptide enzyme inhibitors of the formula:

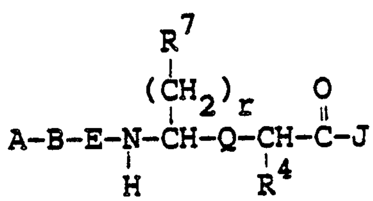

which have N-terminal ureas or sulfamides incorporating polar groups, and analogs thereof, which inhibit renin and are useful for treating various forms of renin-associated hypertension and congestive heart failure; compositions containing these renin-inhibitory peptides, optionally with other antihypertensive agents; and methods of treating hypertension or congestive heart failure or of establishing renin as a causative factor in these problems which employ the novel tripeptides.

EP 0 314 239 A2

# TRIPEPTIDE RENIN INHIBITORS WITH N-TERMINAL UREIDO OR SULFAMIDO GROUPS

The present invention is concerned with novel peptides having N-terminal ureas or sulfamides incorporating polar groups, which inhibit the angiotensinogen cleaving action of the proteolytic enzyme, renin, with pharmaceutical compositions containing the novel peptides of the present invention as active ingredients, with methods of treating renin-associated hypertension, hyperaldo steronism and congestive heart failure, with diagnostic methods which utilize the novel peptides of the present invention, and with methods of preparing the novel peptides of the present invention.

## BACKGROUND OF THE INVENTION

Renin is an aspartic proteinase (molecular weight about 40,000) produced and secreted by the juxtaglomerular cells of the kidney. Renin has a high specificity for and cleaves the naturally-occurring plasma glycoprotein, angiotensinogen, at only the 10, 11 peptide bond, i.e., between the 10th (Leu) and 11th (Leu) amino acid residues in the equine substrate, as described by Skeggs et al, J. Exper. Med. 1957, 106, 439, or between Leu 10 and Val 11 in the human renin substrate, as elucidated by Tewksbury et al, Circulation 59, 60, Supp. II. 132, Oct. 1979.

This cleavage of its protein substrate, angiotensinogen, by the endopeptidase, renin, splits off the decapeptide, angiotensin I, which itself is thought to be hemodynamically-inactive, but which is converted in the lungs, kidneys or other tissue by the peptidase, angiotensin-converting enzyme (ACE), to the potent vasopressor octapeptide, angiotensin II. Thus released, the hormone, angiotensin II, then causes constriction of the arterioles and is also believed to stimulate release of the sodium-retaining hormone, aldosterone, from the adrenal cortex, thereby causing a rise in extracellular fluid volume. Accordingly, the renin-angiotensin system plays an important role in normal cardiovascular homeostasis and in some forms of elevated blood pressure (hypertension).

Inhibitors of angiotensin I converting enzyme have proven useful in the modulation of the renin-angiotensin system. Consequently, specific inhibitors of the catalytic and rate-limiting enzymatic step that ultimately regulates angiotensin II production, the action of renin on its substrate, have also been sought as effective investigative tools, as well as therapeutic agents in the treatment of hypertension and congestive heart failure.

Renin antibody, pepstatin, phospholipids, and substrate analogs, including tetrapeptides and octa- to tridecapeptides, with inhibition constants ($K_i$) in the $10^{-3}$ to $10^{-6}$M region, have been studied.

Umezawa et al., in J. Antibiot. (Tokyo) 23: 259-262, 1970, reported the isolation of a peptide, pepstatin, from actinomyces that was an inhibitor of aspartyl proteases such as pepsin, cathepsin D, and renin. Gross et al., Science 175:656, 1972, reported that pepstatin reduces blood pressure in vivo after the injection of hog renin into nephrectomized rats. However, pepstatin has not found very wide application as an experimental agent because of its limited solubility and its inhibition of a variety of other acid proteases in addition to renin.

Many efforts have been made to prepare a specific renin inhibitor based on pig renin substrate analogy, since such analogy has been shown to correlate well with and predict human renin inhibitor activity. The octapeptide amino acid sequence extending from histidine-6 through tyrosine-13

```
  6   7   8   9   10  11  12  13
(-His-Pro-Phe-His-Leu-Leu-Val-Tyr-)
```

has been shown to have kinetic parameters essentially the same as those of the full tetradecapeptide renin substrate.

Kokubu et al., Biochem. Pharmacol., 22, 3217-3223, 1973, synthesized a number of analogs of the tetrapeptide found between residues 10 to 13, but while inhibition could be shown, inhibitory constants were only of the order of $10^{-3}$M. Analogs of a larger segment of renin substrate have been also synthesized, e.g., Burton et al., Biochemistry 14: 3892-3898, 1975, and Poulsen et al., Biochemistry 12: 3877-3882, 1973, but a lack of solubility and weak binding (large inhibitory constant) generally resulted.

Modifications to increase solubility soon established that the inhibitory properties of the peptides are markedly dependent on the hydrophobicity of various amino acid residues. These modifications also

established that increasing solubility by replacing lipophilic amino acids with hydrophilic isosteric residues can become counter-productive. Other approaches to increasing solubility have also had limited success.

Modifications designed to increase binding to renin have also been made, but here too, with mixed results.

A series of inhibitors of renin have been disclosed which contain the unnatural amino acid, statine or its analogs: see, e.g., Veber et al, U.S. Patents 4,384,994 and 4,478,826; Evans et al, U.S. Patent 4,397,786; Boger et al, Nature, 1983, 303, 81-84 and U.S. Patents 4,470,971; 4,485,099; 4,663,310 and 4,668,770; Matsueda et al, EP-A 128 762, 152 255; Morisawa et al., EP-A 186 977; Riniker et al, EP-A 111 266; Bindra et al, EP-A 155 809; Stein et al, Fed. Proc. 1986, 45, 869; and Hölzemann et al, German Offenlequngsschrift DE 3438545. Attempting to explain the effect of statine, Powers et al., in Acid Proteases, Structure, Function and Biology, Plenum Press, 1977, 141-157, observed that in pepstatin, statine occupies the space of the two amino acids on either side of the cleavage site of a pepsin substrate and Tang et al., in Trends in Biochem. Sci., 1:205-208 (1976) and J. Biol. Chem., 251:7088-94, 1976, suggested that the statine residue of pepstatin resembles the transition state for pepsin hydrolysis of peptide bonds.

Renin inhibitors containing other peptide bond isosteres, including a reduced carbonyl isostere, have been disclosed by M. Szelke et al, in work described in published European Patent Applications 45 665 and 104 041; in U.S. Patent 4,424,207, and in PCT Int. Appl. WO 84/03044; in Nature, 299, 555 (1982); Hypertension, 4, Supp. 2, 59, 1981; and British Patent 1,587,809. In Peptides, Structure and Function: Proceedings of the Eighth American Peptide Symposium, ed. V. J. Hruby and D. H. Rich, p. 579, Pierce Chemical Co., Rockford, IL., 1983, Szelke et al also showed isosteric substitutions at the Leu-Leu site of cleavage, resulting in compounds with excellent potency. These and other peptide bond isosteres have also been disclosed in Buhlmayer et al in EP-A 144 290 and 184 550; Hester et al, EP A 173 481; Raddatz, EP-A 161 588; Dann et al, Biochem. Biophys. Res. Commun. 1986, 134, 71-77; Fuhrer et al. EP-A 143 746; Kamijo et al, EP-A 181 110; Thaisrivongs et al, J. Med. Chem., 1985, 28, 1553-1555; Ryono et al., EP-A 181 071; and Evans et al, U.S. patent 4,609,641.

Other modifications which have been tried include preparing renin inhibitors with non-peptide C-termini, such as disclosed in European Published Applications 172 346 and 172 347; Evans et al, J. Med. Chem., 1985, 28, 1755-1756,. and Bock et al, Peptides, Structure and Function: Proceedings of the Ninth American Peptide Symposium, ed. C. M. Deber et al, pp.751-754, Pierce Chemical Co., Rockford, IL, 1985. Kokubu et al, in Hypertension, 1985, 7, Suppl. I, p. 8-10 and Matsueda et al, in Chemistry Letters, 1985, 1041-1044 and in European Published Applications 128 762 and 152 255 disclosed peptide aldehyde renin inhibitors, and Hanson et al in Biochem. Biophys. Res. Commun. 1985, 132, 155-161, reported peptide glycol inhibitors.

Modifications at the N-terminii, suggesting the use of ureas include Raddatz et al, EPO Published Application 220 665, wherein several tripeptides were shown with urea-containing amino terminii, and Morisawa et al, EPO Published Application 228 192, which broadly suggested ureas at the amino-terminus. Acyclic ureas and sulfamides have been covered generically by Buhlmayer et al in EPO-A 184 550, cited above, wherein the peptide bond isostere is Cal[CH(OH)$CH_2$]Val, and ureas and sulfamides at the N-terminus in peptides wherein the carboxy-terminus contains diol-type structures have been disclosed in Luly et al EPO-A 229 667.

These various renin inhibitors all generally comprise peptide based inhibitors in which a sequence of the type: ...A-B-D-E-F-G-J-K-L... , where G is a peptide bond mimic and A,B,D,E,F,J,K, and L may individually be absent or may represent naturally-occuring or modified amino acids. Typical sequences of this type include:

```
                     7   8   9   10  11  12
this type include: ...BOC-Pro-Phe-His-Sta-Leu-Phe... ,

              8   9   10  11
or  ...BOC-Phe-His-Sta-Leu... ,
```

where the N-terminus typically comprises an amino acid protecting group such as BOC or CBZ, and the N-terminal amino acids are Pro-Phe-His or Phe-His.

It was an object of this invention to prepare potent, lower molecular weight renin-inhibitory peptides which have improved water solubility and oral bioavailability. It was a further object of this invention to prepare peptides comprising modifications at the C- and N-terminii of a shortened peptide. It was an additional object of this invention to prepare peptides in which polar and/or charged groups (+ and/or -)

have been selectively introduced at strategic positions in order to constructively alter the physical properties of these peptides. It was still a further object of this invention to prepare novel peptides which more effectively inhibit the activity of renin and thereby are more useful antihypertensive agents, and are also compounds useful in treating congestive heart failure.

## DETAILED DESCRIPTION OF THE INVENTION

The present invention discloses renin-inhibitory tripeptides having amine-containing ureas at the N-terminii, which have the formula:

A-B-E-G-J,

wherein:

A is

hydrogen;

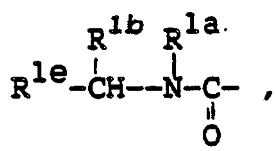

where

$R^{1a}$ and $R^{1b}$ are independently hydrogen or $C_1$-$C_4$ alkyl or $R^{1a}$ and $R^{1b}$taken together are $-(CH_2)_3-$;

$R^{1e}$ is hydrogen; aryl, wherein aryl is unsubstituted or mono-, di or trisubstituted phenyl or naphthyl, where the substituent(s) is/are independently selected from the group consisting of $C_1$-$C_8$-alkyl, amino, mono- or di-$C_1$-$C_4$-alkylamino, amino-$C_1$-$C_4$-alkyl, hydroxy-$C_1$-$C_4$-alkyl, phenyl-$C_1$-$C_4$-alkyl, mono-or di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2$-$C_1$-$C_4$-alkyl, $CO_2$-$C_1$-$C_4$-alkoxy, $NR^5R^6$, and $N(R^5)_3^{\oplus} A^{\ominus}$, wherein $R^5$ and $R^6$ are independently hydrogen, unsubstituted or monosubstituted $C_1$-$C_4$-alkyl, where the substituent is amino, mono- or di-$C_1$-$C_4$-alkylamino, hydroxyl, $C_1$-$C_4$-alkoxy or $N(C_1\text{-}C_4\text{-alkyl})_3^{\oplus} A^{\ominus}$; and $A^{\ominus}$ is a counterion selected from the group consisting of small, single negatively-charged ions, such as chloride, bromide, nitrate, perchloride, benzoate, maleate, benzene sulfonate, tartrate, hemitartrate and acetate; Het, wherein Het is an unsubstituted or mono- or disubstituted 5- to 7-membered mono- or bicyclic or 7- to 10-membered bicyclic heterocyclic ring, where the one or two heteroatoms are independently selected from the group consisting of N, O, S, NO, SO, $SO_2$ and quaternized N, and the substituent(s) is/are independently selected from the group consisting of hydroxyl, thiol, $C_1$-$C_6$-alkyl, $CF_3$, $C_1$-$C_4$-alkoxy, halo, aryl, as defined above, aryl-$C_1$-$C_4$-alkyl, amino, mono- or di-$C_1$-$C_4$-alkylamino, amino-$C_1$-$C_4$-alkylamino, amino-$C_1$-$C_4$-alkyl, hydroxy-$C_1$-$C_4$-alkyl, di-$C_1$-$C_4$-alkyl-amino-$C_1$-$C_4$-alkyl, guanidyl, guanidyl-$C_1$-$C_4$-alkyl, CHO, $CO_2H$, $CO_2$-$C_1$-$C_4$-alkyl, $NR^5R^6$, and $N(R^5)_3^{\oplus} A^{\ominus}$ wherein $R^5$, $R^6$ and $A^{\ominus}$ are as defined above, or alternatively when N is present as a heteroatom, the substituents are $-(CH_2)_q-$ or $-(CH_2)_2-O-(CH_2)_2$ and form a quaternary spirocyclic ring with the N atom, wherein q is 3-to-6; $-CO_2R^{1a}$ or $-CONR^{1a}R^{1b}$ wherein $R^{1a}$ and $R^{1b}$ are as defined above; $C_3$-$C_8$-cycloalkyl; $-CONHSO_2$-aryl, wherein aryl is as defined above; or unsubstituted or mono- or disubstituted $C_1$-$C_4$-alkyl, wherein the substituent(s) is/are independently selected from the group consisting of $C_3$-$C_7$-cycloalkyl; Het, as defined above; aryl, as defined above; $-CO_2R^{1a}$ or $-CONR^{1a}R^{1b}$, where $R^{1a}$ and $R^{1b}$ are as defined above; $-CONHSO_2$-aryl, where aryl is as defined above, $R^{1c}R^{1d}N-$, where $R^{1c}$ and $R^{1d}$ are independently hydrogen; unsubstituted or monosubstituted $C_1$-$C_4$-alkyl, where the substituent is $CO_2R^{1a}$ or $CONR^{1a}R^{1b}$, wherein $R^{1a}$ and $R^{1b}$ are as defined above;

aryl-$C_1$-$C_4$-alkyl, wherein aryl is as defined above; $-CONR^5R^6$, where $R^5$ and $R^6$ are as defined above; or unsubstituted or monosubstituted $C_2$-$C_4$-alkyl, wherein the substituent is on the terminal carbon atom and is -OH or $-NR^{1a}R^{1b}$, where $R^{1a}$ and $R^{1b}$ are as defined above; -OH; $-SO_3H$; quanidino, $-CO_2H$; $-CO_2$-$C_1$-$C_4$-alkyl; and $-CO-NH-SO_2$-aryl;

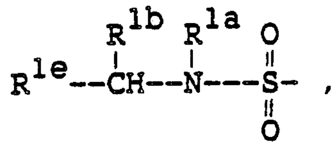

where
$R^{1a}$, $R^{1b}$, $R^{1c}$, $R^{1d}$ and $R^{1e}$ are as defined above;

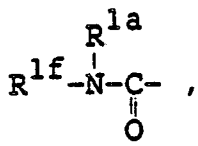

where
$R^{1f}$ is aryl, as defined above, or Het, as defined above; and $R^{1a}$ is as defined above;

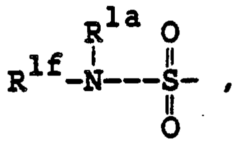

where
$R^{1f}$ and $R^{1a}$ are as defined above;

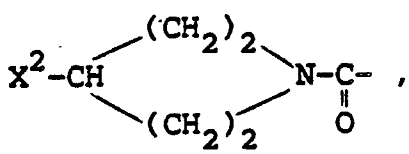

where
$X^2$ is O, S, SO, $SO_2$, N-$R^{1c}$, wherein $R^{1c}$ is as defined above;

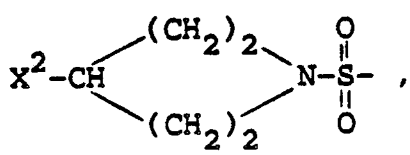

where
$X^2$ is as defined above;

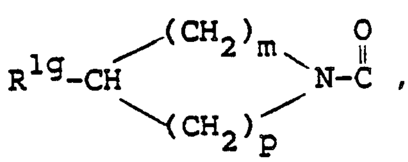

where
m and p are independently 1 or 2; and

$R^{1g}$ is hydroxyl; $-NR^{1c}R^{1d}$, wherein $R^{1c}$ and $R^{1d}$ are as defined above; or any of the definitions of $R^{1e}$, as listed above;

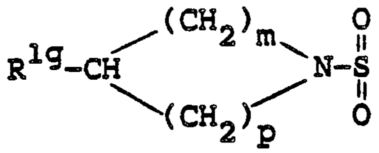 ,

where

$R^{1g}$, m and p are as defined above;

B is

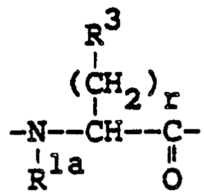 ,

where

r is 1-to-4;

$R^3$ is hydrogen; $C_1$-$C_4$-alkyl; aryl, as defined above; or indolyl; and

$R^{1a}$ is as defined above;

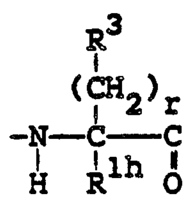

where

$R^{1h}$ is $C_1$-$C_4$-alkyl; and

$R^3$ and r are as defined above; or

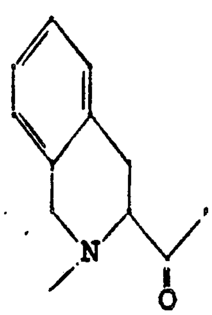 ;

E is

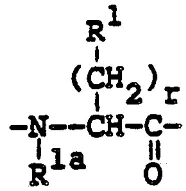 ,

where
r is as defined above; and
$R^1$ is hydrogen; aryl, as defined above; Het, as defined above; or unsubstituted or mono- or disubstituted $C_1$-$C_4$-alkyl, wherein the substituent(s) is/are on the terminal carbon atom(s) and is/are chosen from the group consisting of $-CO_2R^{1a}$ and $-CONR^{1a}R^{1b}$, where $R^{1a}$ and $R^{1b}$ are as defined above; and when r is 2, 3 or 4, alternatively $C_1$-$C_4$-alkoxy, mono- or di-$C_1$-$C_4$-alkyl, $-NH_2$, and -OH; or

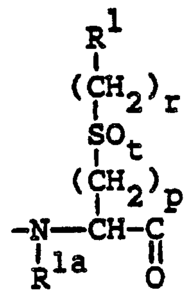 ,

where
t is 0-to-2; and
$R^1$, $R^{1a}$, p and r are as defined above;
G is

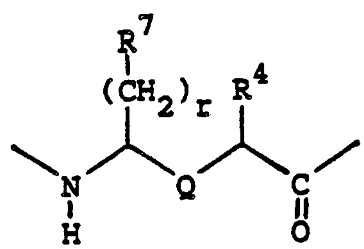 ,

where
$R^4$ is hydrogen; $C_1$-$C_8$-alkyl;p $C_2$-$C_8$-alkenyl; mono- or disubstituted $C_2$-$C_8$-alkyl, wherein the substituent(s) is/are on the final 1 or/and 2 carbon atoms of the alkyl chain and is/are independently selected from the group consisting of hydroxyl, carboxy, $CO_2$-$C_1$-$C_4$-alkyl, amino, mono-, di-, or tri-$C_1$-$C_4$-alkylamino, guanidyl, and $NR^5R^6$ and $N(R^5)_3{}^{\oplus}A^{\ominus}$, wherein $R^5$, $R^6$ and $A^{\ominus}$ are as defined above; aryl, as defined above; unsubstituted or mono-, di-or trisubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy, and halo;
$R^7$ is hydrogen; $C_3$-$C_6$-alkyl; aryl, as defined above; or unsubstituted or mono-, di- or trisubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy, and halo;
Q is

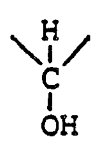 ; 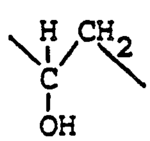 ; 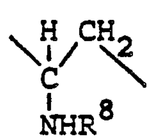 or 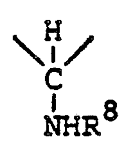

wherein
$R^8$ is H; $C_1$-$C_4$-alkyl; $C_1$-$C_4$-alkanoyl; aroyl; $C_1$-$C_4$-alkoxycarbonyl; aryloxycarbonyl; or aryl-$C_1$-$C_4$-alkoxycarbonyl, where aryl is as defined above; and
r is as defined above; and

J is
$-Y-(CH_2)_n-R^{10}$,
where
n is 0-to-5; and
Y is -NH-, -O- or $N(CH_2)_n-R^{10}$, wherein $R^{10}$ is hydrogen, hydroxy; $C_1$-$C_4$-alkyl; $C_3$-$C_7$-cycloalkyl; $C_1$-$C_4$-alkyl-$SO_2OH$; aryl, as defined above; $NR^{11a}R^{11b}$, wherein $R^{11a}$ and $R^{11b}$ are independently hydrogen; unsubstituted or mono-substituted $C_1$-$C_4$-alkyl, wherein the substituent is carboxy, sulfo or hydroxy; monosubstituted $C_2$-$C_4$-alkyl, wherein the substituent is on the terminal carbon and is amino or mono- or di-$C_1$-$C_4$-alkylamino; aryl, as defined above; aryl-$C_1$-$C_4$-alkyl; Het, as defined above; Het-$C_1$-$C_4$ alkyl, $N(R^{11a})_3^{\oplus} A^{\ominus}$, where $R^{11a}$ and $A^{\ominus}$ are as defined above; guanidino-$C_2$-$C_4$-alkyl; or guanidyl; except that when n is 0 or 1, $R^{10}$cannot be hydroxy or $NR^{11a}R^{11b}$;

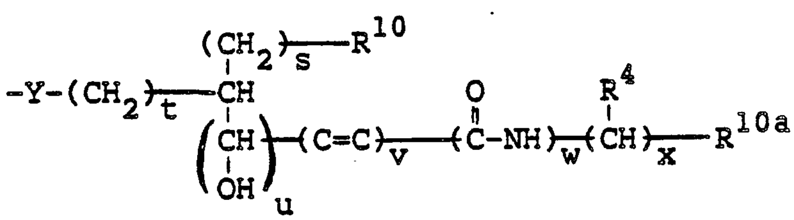

where
$R^{10a}$ is independently one of the definitions of $R^{10}$,

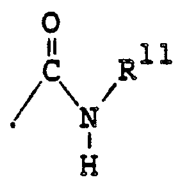

or

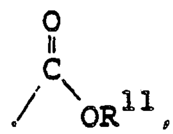

where $R^{11}$ is hydrogen or $C_1$-$C_3$-alkyl;
x is 1 to 4;
t, u, v and w are independently 0 or 1, provided that w is not 1 when v is 0 and when t is 0, u, v, w and x are not simul taneously 0; and
Y, $R^4$, $R^{10}$ and s are as defined above;

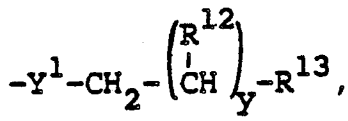

where
$Y^1$ is -NH-, -NH-, -N-$C_1$-$C_4$-alkyl- or -O-;
Y is 1 to 4;
$R^{12}$ is hydrogen; hydroxy; $NH_2$; $N(C_1$-$C_4$-alkyl$)_2$; $NH(C_1$-$C_4$-alkyl); guanidyl; or $N(C_1$-$C_4$-alkyl$)_3^{\oplus} A^{\ominus}$, wherein $A^{\ominus}$ is as defined above;
provided that at least one $R^{12}$ is other than hydrogen; and
$R^{13}$ is $C_1$-$C_4$-alkyl; $C_3$-$C_7$-cycloalkyl; aryl, as defined above, with the substituent(s) on the substituted aryl also alternatively selected from the group consisting of α-aminocarboxy-$C_1$-$C_4$-alkyl, α-amino-carboxy-$C_1$-$C_4$-alkyl ester or amide, and carboxy-$C_1$-$C_4$-alkyl ester or amide, $CO_2H$; $CO_2$-$C_1$-$C_4$-alkyl; $NR^5R^6$, wherein $R^5$ and $R^6$ are defined as above; sulfo; or Het, as defined above;

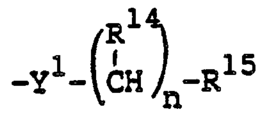,

where

$R^{14}$ is hydrogen, $CO_2H$, $CO_2$-$C_1$-$C_4$-alkyl or $NR^5R^6$, wherein $R^5$ and $R^6$ are as defined above;

$R^{15}$ is $CO_2H$; $CO_2$-$C_1$-$C_4$-alkyl; $NR^5R^6$, wherein $R^5$ and $R^6$ are as defined above; sulfo; or aryl, as defined above, with the substituent(s) on the substituted aryl also alternatively being selected from the group consisting of α-aminocarboxy-$C_1$-$C_4$-alkyl, α-aminocarboxy-$C_1$-$C_4$alkyl ester or amide, and carboxy-$C_1$-$C_4$alkyl ester or amide, or when n is 1, $R^{15}$ is alternatively $C_1$-$C_4$-alkyl, $C_3$-$C_7$-cycloalkyl or Het, as defined above; and

$Y^1$ and n are defined as above;

except that when n is 1, neither $R^{14}$ nor $R^{15}$ is $NR^5R^6$, when n is 1 and $R^{14}$ is H, $R^{15}$ cannot be $CO_2H$ or $CO_2$-$C_1$-$C_4$-alkyl, when n is 1 and $R^{14}$ is $CO_2H$ or $CO_2$-$C_1$-$C_4$-alkyl, $R^{15}$ cannot be aryl, and when n is 2, 3, 4 or 5, the $R^{14}$ on the carbon adjacent $Y^1$ cannot be $NR^5R^6$;

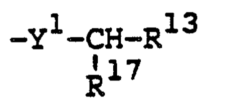,

where

$R^{17}$ is H or $C_1$-$C_4$-alkyl; and

$Y^1$ and $R^{13}$ are as defined above, except that $R^{13}$ in this formula cannot be $NR^5R^6$, $CO_2H$ or $CO_2$-$C_1$-$C_4$-alkyl;

or

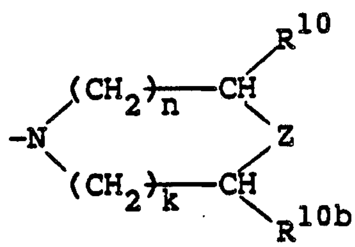

where

Z is NH, $NR^{10}$, $NR^{11a}R^{11b}$, O, S, SO, $SO_2$ or $CHR^{10}$, wherein $R^{10}$ is as defined above;

k is 1-to-5; and

$R^{10b}$ is independently one of the definitions of $R^{10}$; Het, as defined above; or Het substituted by a 5- or 6-membered heterocyclic ring or benzofused heterocyclic ring, where the one or two heteroatoms are independently selected from N, O and S;

and pharmaceutically acceptable salts thereof.

In the peptides of the present invention, the A, B, E, G and J components have asymmetric centers and occur as racemates, racemic mixtures and as individual diastereomers, with all isomeric forms generally being included in the present invention. In particular, asymmetric carbon atoms in the G substituent has either an S or an R configuration, except as noted in one alternate definition of G above, and all other asymmetric carbon atoms have an S configuration, with preferred chiralities indicated in the following further description.

When any variable (e.g., aryl, Het, $R^{1a}$, $R^{1b}$, $R^5$, $R^6$, $R^7$, $A^{\ominus}$, r, etc.) occurs more than one time in any constituent or in formula I, its definition on each ocurrence is independent of its definition at every other occurrence.

As used herein, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge; and "$C_3$-$C_7$-cycloalkyl" is intended to include saturated ring groups, such as cyclopropyl,

cyclobutyl, cyclopentyl, cyclohexyl (Cyh) and cycloheptyl. "Alkanoyl" is intended to include those alkylcarbonyl groups of specified number of carbon atoms, which are exemplified by formyl, acetyl, propanoyl and butanoyl; "alkenyl" is intended to include hydrocarbon chains groups of either a straight- or branched-configuration and one unsaturation, which may occur at any point along the chain, such as ethenyl, propenyl, butenyl, pentenyl, and the like, and includes E and Z forms, where applicable; and "arylalkyl" represents aryl groups as herein defined which are attached through a straight-or branched- chain alkyl group of specified number of carbon atoms, such as, for example, benzyl, phenethyl, 3,3-diphenylpropyl, 3-indolymethyl, and the like. "Halo", as used herein, means fluoro, chloro, bromo and iodo, and "counterion" is used to represent a small, single negatively-charged specie, such as chloride, bromide, hydroxide, acetate, perchlorate, benzoate, amleate, benzene sulfonate, tartrate, hemitartrate, and the like.

As used herein, with exceptions as noted, "aryl" is intended to mean phenyl (Ph) or naphthyl, which is optionally-substituted by from one- to three- members independently selected from the group consisting of amino (Am), mono- or di-$C_1$-$C_4$-alkyl-amino, amino-$C_1$-$C_4$-alkyl, hydroxy-$C_1$-$C_4$-alkyl, phenyl-$C_1$-$C_4$-alkyl, di-$C_1$-$C_4$-alkylamino- $C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2R^7$, $NR^5R^6$, wherein $R^7$, $R^5$ and $R^6$ are as defined above, or $N(R^5)\overset{\oplus}{_3}A^{\ominus}$, wherein $R^5$ is as defined above an a counterion, as defined herein. "Aroyl" is intended to include those aryl carbonyl groups which are exemplified by benzoyl and naphthoyl.

The term "Het", as used herein, represents a 5- to 7- membered mono- or bicyclic or 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. Fully-saturated heterocyclic substituents are preferred and those in which nitrogen is the heteroatom are also preferred, with those containing a single nitrogen atom being particularly preferred. The heterocyclic ring may be attached at any heteroatom or carbon atom which results in the creation of a stable structure. Examples of such heterocyclic elements include piperidyl, piperidinyl, piperazinyl, 2-oxopiperazinyl, 2-oxopiperidinyl, 2-oxopyrrolodinyl, pyrryl, pyrrolinyl, 4-piperidonyl, pyrrolidinyl, pyrazolyl, pyrazolinyl, pyrazolidinyl, imidazolyl, imidazolinyl, imidazolidinyl, pyridyl, pyrazinyl, pyrimidinyl, pyridazinyl, oxaolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, benzothiazolyl, benzoxazolyl, furyl, thienyl, benzothienyl,. azepinyl, 2-oxoazepinyl, thiamorpholinyl, thiamorpholinyl sulfoxide, and thiamorpholinyl sulfone. The heterocyclic moiety is further optionally-substituted by from one- to three-members independently selected from the group consisting of hydroxyl, thiol, $C_1$-$C_6$-alkyl, $CF_3$, $C_1$-$C_4$-alkoxy, halo, aryl, aryl-$C_1$-$C_4$-alkyl, amino, mono-or di-$C_{1-4}$-alkylamino, amino-$C_1$-$C_4$-alkylamino, amino-$C_1$-$C_4$-alkyl, hydroxy-$C_1$-$C_4$-alkyl, $C_1$-$C_4$-dialkylamino-$C_1$-$C_4$-alkyl, guanidyl, guanidyl $C_1$-$C_4$-alkyl, hydroxyl, CHO, $CO_2H$, $CO_2R^7$, $NR^5R^6$, wherein $R^7$, $R^5$ and $R^6$ are as defined above, or $N(R^5)\overset{\oplus}{_3}A^{\ominus}$, wherein $R^5$ is as defined above is $A^{\ominus}$ is a counterion, as defined herein, with aryl-$C_1$-$C_4$-alkyl being preferred, or alternatively a spiro quaternary ammonium bicyclic species may be formed.

The following additional abbreviations have also been used herein:

| Abbreviated Designation | Amino Acid/Residue |
|---|---|
| ACHPA | (3<u>S</u>,4<u>S</u>)-4-amino-5-cyclohexyl-3-hydroxypentanoic acid |
| Ala | D- or L-alanine |
| Arg | D- or L-arginine |
| Cal (Cha) | ß-cyclohexylalanine |

| | |
|---|---|
| Cys | D- or L-cysteine |
| Gly | glycine |
| His | D- or L-histidine |
| HPhe (HomoPhe) | D- or L-homologated phenylalanine |
| HomoACHPA | homologated ACHPA |
| Ile | D- or L-isoleucine |
| Leu | D- or L-leucine |
| Lys | D- or L-lysine |
| Met | D- or L-methionine |
| Nle | D- or L-norleucine |
| Nva | D- or L-norvaline |
| Orn | D- or L-ornithine |
| Phe | D- or L-phenylalanine |
| Ser | D- or L-serine |
| Sta | statine, (3S,4S)-4-amino-3-hydroxy-6-methylheptanoic acid |
| Thr | D- or L-threonine |
| Trp | L-tryptophan |
| Tyr | L-tyrosine |

Protecting Group

| | |
|---|---|
| BOC (Boc) | t-butyloxycarbonyl |
| BOM | benzyloxymethyl |
| CBZ (Cbz) (Z) | benzyloxycarbonyl(carbobenzoxy) |
| DNP | 2,4-dinitrophenyl |
| EtOC | ethoxycarbonyl |
| IPOC | isopropoxycarbonyl |
| OMe | methoxy, except when it immediately follows an amino acid residue abbreviation, and it represents methyl ester |
| TBDMS | t-butyldimethylsilyl |

| | |
|---|---|
| | Activating Group |
| HBT(HOBt) | N-hydroxybenzotriazole |
| | Condensing Agent |
| DCCI (DCC) | N,N'-dicyclohexylcarbodiimide |
| | Reagent |
| $Et_3N$ (TEA) | triethylamine |
| i-$Pr_2NEt$ | diisopropylethylamine |
| TFA | trifluoroacetic acid |
| | Coupling Reagents |
| BOP reagent | benzotriazol-1-yloxytris(dimethylamino)phosphonium hexafluorophosphate |
| BOP-Cl | bis(2-oxo-3-oxazolidinyl)phosphinic cloride |
| | Solvent |
| AcOH (HOAc) | acetic acid |
| $CH_2Cl_2$ | methylene chloride |
| DMF | dimethylformamide |

The novel renin inhibitory peptides of the present invention may alternately be described in terms of common amino acid components and closely-related analogs thereof, wherein, in accordance with the usages of formula I:

A, G and J are as defined under Formula I;
B is Ala, Leu, Phe, HomoPhe, BisHomoPhe, Tyr, HomoTyr, Trp, or HomoTrp; and
E is Ala, Leu, Ser, Thr, Phe, Tyr, Trp, His, (N methyl)His, Lys, Orn, Arg, Cys, Nle, (N methyl)Nle, Nva, or Met.

In the peptides of the instant invention, the double amino acid sequence of the endogenous human substrate, $Leu^{10}$-$Val^{11}$ is replaced by a G component statine analog/residue (particularly ACHPA or Cal[CH-(OH)$CH_2$]Val). This substitution results in an increase in metabolic stability and a better fit to the renin enzyme.

Also in these novel shortened peptides, amino terminal urea moieties containing polar and/or charged groups are incorporated, which, due to the contribution probably of both the polar and/or charged groups and the urea moiety, substantially improve water solubility and oral bioavailability of the renin inhibitory peptides. These peptides are therefore of lower molecular weight and different structure, with an enhanced potency and duration of action, than known peptides of this class.

It will be understood that closely-related analogs of the above common amino acids, for example, aliphatic amino acids in addition to Ala, Val, Leu, and Ile, such as $\alpha$-aminobutyric acid (Abu), and substituted phenyl derivatives of Phe, are included in the broad description of the novel inhibitory peptides of the present invention represented by Formula I and related definitions.

Representative preferred renin-inhibitory peptides of the present invention include the following:

N-(2 Diethylamino)ethylcarbamoyl)Phe-His-ACHPA-NH-2(S) methylbutyl;
N-(Methyl-2-(morpholin-4-yl)ethylcarbamoyl)Phe-His-ACHPA-NH-2(S)-methylbutyl;
N-(Methyl-3-(morpholin-4-yl)propylcarbamoyl)Phe-His-ACHPA-NH-2(S)-methylbutyl;
N-(Methyl-(pyridin-4 yl)methylcarbamoyl)Phe-His ACHPA-NH-2(S)-methylbutyl;
N-(Methyl-(p-carboxyphenyl)methylcarbamoyl)Phe-His-ACHPA-NH-2(S)-methylbutyl;
N-(Methyl-3-carboxypropylcarbamoyl)Phe-His-ACHPA-NH-2(S)-methylbutyl;
N-(Methyl-3-sulfonylpropylcarbamoyl)Phe-His-ACHPA-NH-2(S) methylbutyl;
N-((1-(2 Butyl)-2 diethylamino)ethylcarbamoyl)Phe-His-ACHPA-NH-2(S) methylbutyl;
N-((1-(2 Propyl) 2-diethylamino)ethylcarbamoyl)-Nα-$CH_3$)Phe-His-ACHPA-NH-2(S) methylbutyl;
N-((1-(Isobutylpyrrolidin-3-yl)carbamoyl)Phe-His-ACHPA-NH-2(S)-methylbutyl;
N-((1-(2-Hydroxy)ethylpyrrolidin-3-yl)carbamoyl)Phe-His-ACHPA-NH-2(S)-methylbutyl;
N-((1-(Carboxymethylpyrrolidin-3-yl)carbamoyl)-(α-$CH_3$)Phe-His-ACHPA-$NHCH_2CH_2N(CH_2CH_2)_2O$;
N-((1-Ethylpiperidin-4-yl)carbamoyl)Phe-His-ACHPA-NH-2(S)-methylbutyl;
N-((1-(2-Butyl)-2-(morpholin-4-yl)ethylcarbamoyl)Phe-His-ACHPA-NH-2(S)-methylbutyl;
N-((1-(2-Butyl)-2-(4-methylpiperazin-1-yl))ethyl-carbamoyl)Phe-His-ACHPA-NH-2(S)-methylbutyl;
N-((1-(2-Butyl)-2-(thiamorpholinesulfon-4-yl))ethyl-carbamoyl)Phe His ACHPA-NH-2(S) ;
N-(2-(Morpholin-4-yl)ethylcarbamoyl)Phe-His-ACHPA NH-2(S)-methylbutyl;
N-(Methyl-2-(morpholin-4-yl)ethylcarbamoyl)-($N^{\alpha}$-$CH_3$)-Phe-His-ACHPA-NH-2(S)-methylbutyl;
N-((Morpholin-4-yl)carbonyl)Phe-His-ACHPA-NHCH(2-butyl)-CH(OH)$CH_2N(CH_2CH_2)_2O$;
N-((1-Methylpiperazin-4-yl)carbonyl)-($N^{\alpha}$-$CH_3$)Phe-His-ACHPA-NH-2(S)-methylbutyl;
N-((Thiamorpholinesulfon-4-yl)carbonyl)Phe-His-ACHPA-NHCH(2-butyl)$CH_2CH_2N(CH_2CH_2)_2O$;
N-((1-carboxymethylpiperazin-4-yl)carbonyl)-(α-$CH_3$)Phe-His-ACHPA-NH-2(S)-methylbutyl;
N-((1-(2-Hydroxy)ethylpiperazin-4-yl)carbonyl)-$N^{\alpha}$-$CH_3$)Phe-His-ACHPA-NH-2(S)-methylbutyl;
N-((1-(2-Dimethylamino)ethylpiperazin-4 yl)carbonyl)-Phe-His-ACHPA-NH-2(S)-methylbutyl;
N-((1-(2-Dimethylcarboxamido)ethylpiperazin-4-yl)-carbonyl)Phe-His-ACHPA-NH-2(S)-methylbutyl;
N-((4-Dimethylaminopiperidin-1-yl)carbonyl)-($N^{\alpha}$-$CH_3$)-Phe-His-ACHPA-NHCH(2-butyl)$CH_2CO_2H$;
N-((3-Diethylaminopiperidin-1-yl)carbonyl)Phe-His-ACHPA-NH-2(S)-methylbutyl;
N-((2-Diethylaminomethylpyrrolidin-1-yl)carbonyl)Phe-His-ACHPA-NH-2(S)-methylbutyl;
N-((Morpholin-4-yl)carbonyl)-($N^{\alpha}$-$CH_3$)Phe-His-Cal-[CH(OH)$CH_2$]Val-NH-$CH_2CH_2N(CH_2)_2O$;
N-(Morpholin-4-yl)carbonyl)Phe-His-Cal[CH(OH)$CH_2$]-Val-NH-i-Bu;
N-((Morpholin-4-yl)carbonyl)Phe-($N^{\alpha}$-CH )His-Cal[CH(OH)$CH_2$]Val-NH-2(S)-methylbutyl;
N-((Morpholin-4-yl)carbonyl)Phe-His-Cal[CH(OH)$CH_2$]-Val-$NHCH_2CH_2N(CH_2CH_2)_2O$;
N-((Morpholin-4 yl)carbonyl)Phe-His-Cal[CH(OH)$CH_2$]-Val-NH-$CH_2$(pyridin-4 yl);
[N-((Morpholin-4-yl)carbonyl)-($N^{\alpha}$-$CH_3$)Phe-His-Cal-[CH(OH)$CH_2$]Val-NH-(N-methyl-3-quinuclidinonium)-]$^{\oplus}$OAc$^{\ominus}$;
N-((Morpholin-4-yl)carbonyl)Phe-($N^{\alpha}$-$CH_3$)His-Cal-[CH(OH)$CH_2$]Val-NH-2(S)-methylbutyl;
N-((Morpholin-4-yl)carbonyl)Phe-His-Cal[CH(OH)$CH_2$]-Val-NH-(3-aminoquinuclidinyl);
N-((Morpholin-4-yl)carbonyl)Phe-His-Cal[CH(OH)$CH_2$]-Val-NH-(N-methyl-3-aminoquinuclidinyl);
N-((Morpholin-4-yl)carbonyl)Phe-His-ACHPA-NH-(3-aminoquinuclidinyl);
[N-((Morpholin-4-yl)carbonyl)Phe-His-ACHPA-NH-(N-methyl-3-aminoquinuclidinyl)]$^{\oplus}$OAc$^{\ominus}$;
N-((4-Methylpiperazin-1-yl)carbonyl)Phe-His-ACHPA-NH-CH(2-butyl)($CH_2N(CH_3)_2$;
[N-((1-Methylpiperazin-4-yl)carbonyl)Phe-His-ACHPA-NH-CH(2-butyl)$CH_2N(CH_3)_3$]$^{\oplus}$OAc$^{\ominus}$;
N-((1-(2-Hydroxy)ethylpiperazin-4-yl)carbonyl)Phe-His-ACHPA-NH-CH(2-butyl)$CH_2N(CH_2CH_2)_2O$;
N-((4-Methylpiperazin-1-yl)carbonyl)Phe-His-ACHPA-NH-CH(2-butyl)$CH_2$NH(4,5-dihydroimidazol-2-yl);
[N-((Morpholin-4-yl)carbonyl)Phe-His-ACHPA-NH-CH(2-butyl)$CH_2N(CH_3)(CH_2CH_2)_2O$]$^{\oplus}$OAc$^{\ominus}$;
N-(2-Diethylamino)ethylsulfamoyl)Phe-His-ACHPA-NH-2(S)-methylbutyl;
N-(Methyl-(2-diethylamino)ethylsulfamoyl)-($N^{\alpha}$-$CH_3$)-Phe-His-ACHPA-NH-2(S)-methylbutyl;
N-((1-(2-Butyl)-2-diethylamino)ethylsulfamoyl)Phe-His-ACHPA-$NH(CH_2)_3N(CH_2CH_2)_2O$;
N-((1-(2-Propyl)-2-diethylamino)ethylsulfamoyl)Phe-His-ACHPA-$NH(CH_2)_3N(CH_2CH_2)_2O$;
N-((1-Isobutylpyrrolidin-3-yl)sulfamoyl)Phe-His-ACHPA-NH-2(S)-methylbutyl;
N-((1-(2-Hydroxy)ethylpyrrolidin-3-yl)sulfamoyl)Phe-His-ACHPA-NH-2(S)-methylbutyl;
N-((Morpholin-4-yl)sulfonyl)-($N^{\alpha}$-$CH_3$)Phe-His-Cal-[CH)OH)$CH_2$]Val-NH-i-Bu;
N-((Morpholin-4-yl)sulfonyl)Phe-($N^{\alpha}$-$CH_3$)Phe-His-Cal-[CH(OH)$CH_2$]Val-NH-2(S)-methylbutyl;
N-((Morpholin-4-yl)sulfonyl)Phe-His-Cal[CH(OH)$CH_2$]-Val-$NHCH_2CH_2N(CH_2CH_2)_2O$;
N-((Morpholin-4-yl)sulfonyl)Phe-($N^{\alpha}$-$CH_3$)His-Cal-[CH(OH)$CH_2$]Val-NH-$CH_2$(pyridin-4-yl);
[N-((Morpholin-4-yl)sulfonyl)-($N^{\alpha}$-$CH_3$)Phe-His-Cal-[CH(OH)$CH_2$]Val-NH-(N-methyl-3-quinuclidinonium)-]$^{\oplus}$OAc$^{\ominus}$;
N-((Morpholin-4-yl)sulfonyl)Phe-($N^{\alpha}$-$CH_3$)His-Cal-[CH(OH)$CH_2$]Val-NH-2(S)-methylbutyl;

N-(Methyl-3-(morpholin-4-yl)propylsulfamoyl)-$N^{\alpha}$-$CH_3$)Phe-His-ACHPA-NH-2(S)-methylbutyl;
N-(Methyl-(pyridin-4-yl)methylsulfamoyl)Phe-His-ACHPA-NHCH(2-butyl)$CH_2N(CH_2CH_2)_2SO_2$;
N-Methyl-(p-carboxyphenyl)methylsulfamoyl)-($N^{\alpha}$-$CH_3$)-Phe-His-ACHPA-NH-2(S)-methylbutyl; and
N-(Methyl-3-carboxypropylsulfamoyl)Phe-His-ACHPA-NH-2(S)-methylbutyl.

The pharmaceutically-acceptable salts of the peptides of Formula I (in the form of water- or oil-soluble or dispersible products) include the conventional non-toxic salts or the quaternary ammonium salts of these peptides, which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

Peptide renin inhibitors of Formula I may be prepared in accordance with well known procedures for preparing peptides from their constitutent amino acids.

Structures and abbreviations for the G components of the renin-inhibitory peptides of the present invention include:

1.

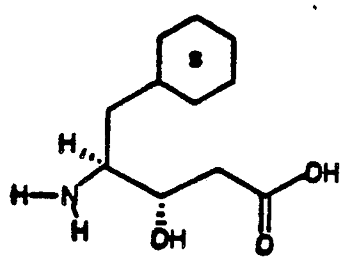

ACHPA

with BOC-ACHPA-OEt being prepared by the method described by Boger et al, J. Med. Chem 1985, 28, 1779-1790;

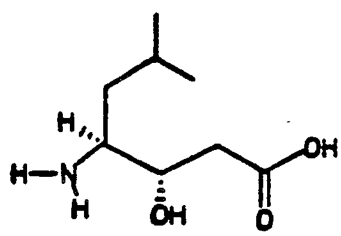

Statine (Sta)

2.

with BOC-Sta-OEt being prepared in accordance with the procedure described by Rich et al, J. Org. Chem., 43, 3624(1978);

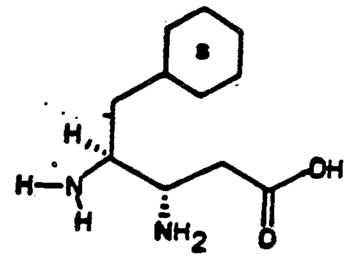

3. AmACHPA

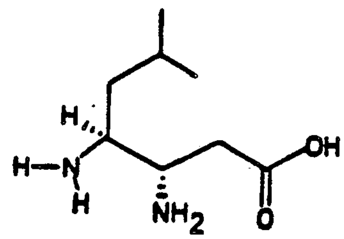

4. AmSta

with BOC-AmSta(CBZ)-OH being prepared as described by Jones et al. in Peptides, Structure and Function. Proceedings of the Ninth American Peptide Symposium (eds. C. M. Deber, V. J. Hruby and K. D. Kopple), pp. 759-62, 1985; Pierce Chemical Co., Rockford, IL; Arrowsmith et al., J. Chem. Soc. Chem. Commun., 755-7 (1986); or Raddatz et al., published EPO Pat. Appln. 161,588,. and BOC-AmACHPA(CBZ)-OH being prepared by similar methods, substituting BOC-ACHPA-OEt for BOC-Sta-OEt;

with efficient methods for preparing the 2-substituted statine component G (such as

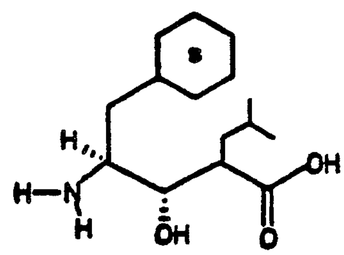

5. (2-isobutyl)ACHPA

with (2 isobutyl)ACHPA and other 2-substituted statine components, in a suitably protected form, being described in Pub. Eur. Pat Appln. 157,409 (with other pertinent references including D. Veber et al, Biochem. Soc. Trans., 12, 956-959 (1984) and H. Stein et al, Fed. Proc. 45, 869, Abstract No 4151 (1986); and

6\. 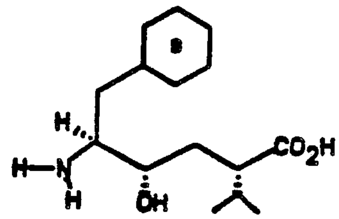

Cal[CH(OH)$CH_2$]Val

with BOC-Cal[CH(OH)$CH_2$]Val-OH lactone being obtained from intermediates prepared using methods described by P. Buhlmayer et al, in Published European Patent Application 184,550-A2, and synthetic routes to similar peptide bond isosteres being described in the following:

a. Szelk et al, in Peptides, Structure and Function. Proceedings of the Eighth American Peptide Symposium (ed. V.J. Hruby and D.H. Rich) pp. 579-82, Pierce Chemical Co., Rockford IL;

b. D.T. Pals et al in European Patent Appln. 173,481-A2;

c. B.E. Evans et al, J. Org. Chem., 50, 4615-1625 (1985);

d. A.H. Fray et al, J.Org. Chem., 51, 4828-4833 (1986);

e. M. Szelke et al, PCT Int. Appl. WO 84 03,044;

f. D.J. Kempf, J. Org Chem., 51, 3921-3926 (1986).

The peptides of the present invention may then be prepared using coupling reactions referred to below in Routes A, B, C and D, including those brought about by the action of DCC/HOBt (dicyclohexylcarbodiimide/N hydroxybenzotriazole), DPPA (diphenylphorylazide), "BOP reagent" (benzotriazol-1-yloxytris(dimethylamino)phosphonium hexafluorophosphate), BOP-Cl (bis(2-oxo-3-oxazolidinyl)phosphinic chloride), DSO (N,N′-disuccinimidyl oxalate), and other well-known coupling reagents.

Peptides of Formula I which contain ACHPA as a peptide bond mimic are prepared using the following procedures or similar procedures (as explained below):

(As used in these routes, the amine components, $R^{2c}R^{2d}NH$, are intended to represent all those encompassed by the definitions of J, including, for example, primary and secondary amines or esters, and the amine components, $R^{2a}R^{2b}NH$, are intended to represent all those encompassed by the definition of A above, also including primary and secondary amines.

Methods used in these procedures for quaternized tertiary amines are well-known and include, for example, that of Chen et al., Can. J. Chem., 54, 3310-11 (1976), and for preparing guanidines from primary and secondary amines, well-known examples include that of Maryanoff, et al., J. Org. Chem., 51, 1882-4 (1986)).

Route A:

Step A1:

BOC-ACHPA-OEt is treated with anhydrous TFA to remove the BOC protecting group, giving ACHPA-OEt;

Step A2:

Using standard methods, a dipeptide protected at the N-terminus with a BOC-group (BOC-$AA^2$-$AA^1$) is coupled to ACHPA-OEt, giving a coupled product 3 or alternatively, BOC-$AA^1$ is coupled to ACHPA-OEt to give product 1. The coupled product 1 is then treated with anhydrous TFA to remove the BOC protecting

group, and the resulting product 2 is coupled with BOC-$AA^2$, to give 3;

Step A3:

The resulting coupled product 3 is treated with methanolic hydrazine, giving the corresponding hydrazide 4;

Step A4:

Hydrazide 4 is treated with acidic isoamyl nitrite, producing the corresponding acyl azide, which is treated in situ with an amine $R^{2c}R^{2d}NH$, giving coupled product 5, or alter natively, coupled product 3 is treated with sodium hydroxide in THF-$H_2O$ and the resulting carboxylic acid derivative coupled with $R^{2c}R^{2d}NH$, to give 5. Additional reactive functional groups in the amino component $R^{2c}R^{2d}NH$ are protected with protecting groups, such as CBZ for amino groups, benzyl ethers for alcohols, and benzyl esters for carboxylic acids;

Step A5:

The N-terminal BOC protecting group is then removed from 5 by treatment with anhydrous TFA, and the resulting tripeptide analog 6 treated with carbonyldiimidazole and shortly thereafter with an amino component, $R^{2a}R^{2b}NH$, to produce the urea 7, or alternatively, the amino component, $R^{2a}R^{2b}NH$, is first treated with carbonyldiimidazole or with phosgene, and the resulting intermediate is allowed to react with 6, to produce the urea 7. Additional reactive functional groups in the amino component, $R^{2a}R^{2b}NH$, are protected with protecting groups, as noted above for $R^{2c}R^{2d}NH$.

Step A5a:

Alternatively, compound 7 may be prepared by coupling compound 2 with $R^{2a}R^{2b}NCO$-$AA^2$, to produce urea 3a, which is then treated with methanolic hydrazine, to give hydrazide 4a. Hydrazide 4a is treated with acidic isoamyl nitrite, to produce the corresponding acyl azide, which in turn, is treated in situ with an amine $R^{2c}R^{2d}NH$, to give compound 7, with additional reactive functional groups in the amino component $R^{2a}R^{2b}NH$ being protected with protecting groups as noted above for $R^{2c}R^{2d}NH$. The $R^{2a}R^{2b}NCO$-$AA^2$ component is prepared by treatment of $AA^2$-O-t-Bu with carbonyldiimidazole and then with $R^{2a}R^{2b}NH$, to give $R^{2a}R^{2b}NCO$-$AA^2$-O-t-Bu, which is treated with anhydrous TFA, giving $R^{2a}R^{2b}NCO$-$AA^2$, which is used as described above.

Step A6:

During the steps above, reactive functional groups present in the side-chains of amino acid components or in the $R^{2c}R^{2d}NH$- element are protected with protecting groups, such as CBZ groups for amines, benzyl ethers for alcohols, and benzyl esters for carboxylic acids, or in the case of histidine, the BOC protecting may be used for protection of the side chain imidazole ring and removed in step A3, during the treatment with hydrazine, or in step A4, during treatment with sodium hydroxide, thereafter, leaving the histidine imidazole ring unprotected;

Step A7:

In cases where a quaternized ammonium group is to be introduced into the C-terminal $R^{2c}R^{2d}NH$- element of compound 8, one of the following procedures is followed:

Procedure 1: A tertiary amine within the $R^{2C}$ or $R^{2d}$ group of $R^{2c}R^{2d}NH$ is quaternized by treatment of $R^{2c}R^{2d}NH$-BOC with an alkyl halide and $KHCO_3$ in methanol or ethanol, or with an alkyl halide in DMF, and the resulting quaternary ammonium salt is treated with anhydrous TFA to remove the BOC protecting group, with the resulting amine used in coupling step A4 above.

Procedure 2: A compound 7, in which the $R^{2c}R^{2d}N$- element contains a tertiary amine, is prepared according to steps A1 to A6, and then the tertiary amine is quaternized by treatment with an alkyl halide in DMF, wherein histidine, when present as the $AA^1$ element, must first be reprotected as the BOC derivative by treatment of 7 with di-t butyldicarbonate, with the BOC group being removed after step A9 by treatment of compound 8 with $K_2CO_3$ in methanol or with anhydrous ammonia in DMF or methanol;

Step A8:

In cases where the $R^{2c}R^{2d}N$-, component of a compound 8 contains a guanidine group, compound 7 is prepared as described in Step A5a, optionally including step A7, with an amine group, present in the $R^{2c}R^{2d}N$- component and hetetofore protected with a BOC group, being liberated by treatment of 7 with anhydrous TFA, and the guanidine group being introduced using standard methods; and

Step A9:

Protecting groups are removed from compound 7 by hydrogenolysis, giving compound 8.

| Compound | Formula |
|---|---|
| 1 | BOC-$AA^1$-ACHPA-OEt |
| 2 | $AA^1$-ACHPA-OEt |
| 3 | BOC-$AA^2$-$AA^1$-ACHPA-OEt |
| 3a | $R^{2a}(R^{2b})N$-CO-$AA^2$-$AA^1$-ACHPA-OEt |
| 4 | BOC-$AA^2$-$AA^1$-ACHPA-$NHNH_2$ |
| 4a | $R^{2a}(R^{2b})N$-CO-$AA^2$-$AA^1$-ACHPA-$NHNH_2$ |
| 5 | BOC-$AA^2$-$AA^1$-ACHPA-$NR^{2c}R^{2d}$ |
| 6 | $AA^2$-$AA^1$-ACHPA-$NR^{2c}R^{2d}$ |
| 7,8 | $R^{2a}(R^{2b})N$-CO-$AA^2$-$AA^1$-ACHPA-$NR^{2c}R^{2d}$ |

Route B:

Step B1:

BOC-ACHPA-OEt is treated with sodium hydroxide in THF-$H_2O$, to give BOC-ACHPA;

Step B2:

BOC-ACHPA-OH is coupled with an amine component $R^{2c}R^{2d}NH$, to give coupled product 9;

Step B3:

Compound 9 is treated with anhydrous TFA to remove the BOC protecting group, to give compound 10;

Step B4:

Compound 10 is coupled with a dipeptide protected at the N-terminus with a BOC protecting group (BOC-$AA^2$-$AA^1$), to give coupled product 12, or alternatively, 10 is coupled with BOC-$AA^1$, to give compound 11, which is treated with anhydrous TFA and the resulting product is coupled with BOC-AA2 to give compound 12;

Step B5:

The N-terminal BOC protecting group is then removed from compound 12 by treatment with anhydrous TFA, and the resulting tripeptide analog 13 is treated first with carbonyldiimidazole and shortly thereafter with an amino component $R^{2a}R^{2b}NH$, to produce the urea 7, or alternatively, the amino component $R^{2a}R^{2b}NH$ is first treated with carbonyldiimidazole or with phosgene, and the resulting intermediate allowed to react with 13, to produce 7;

Step B5a:

Alternatively, compound 11 is treated with anhydrous TFA and the resulting intermediate is coupled to $R^{1a}R^{2b}NCO$-$AA^2$, as described in Step A5a to give compound 7.

Step B6:

In cases where a quaternized amino group is to be introduced into the C-terminal $R^{2c}R^{2d}N$- element of compound 8, one of the following procedures is followed to introduce the quaternized amine group:

Procedure 1: A tertiary amine within the $R^{2C}$ or $R^{2d}$ group of $R^{2c}R^{2d}NH$ is quaternized by treatment of $R^{2c}R^{2d}N$-BOC with an alkyl halide and $KHCO_3$ in methanol or ethanol, or with an alkyl halide in DMF, and the resulting quaternary ammonium salt is treated with anhydrous TFA to remove the BOC protecting group, which results in an amine used in coupling step B2 above;

Procedure 2: A compound 7, in which the $R^{2c}R^{2d}N$- element contains a tertiary amine, is prepared according to steps B1 to B5, and the tertiary amine is quaternized by treatment with an alkyl halide in DMF, or when histidine is present as the $AA^2$ element, it must first be reprotected as the BOC derivative by treatment of 7 with di-t-butyldicarbonate, with the BOC group being removed after step B8 by treatment of compound 8 with $K_2CO_3$ in methanol or with anhydrous ammonia in DMF or methanol; and/or

Step B7:

In cases where the $R^{2c}R^{2d}N$-component of a compound 8 contains a guanidine group, compound 7 is prepared as described in Steps B1 through B5 above (Step B6 being also carried out, if desired), then an

amine group present in the $R^{2c}R^{2d}N$- component and heretofore protected with a BOC group, is liberated by treatment of 7 with anhydrous TFA, with standard methods being used for introduction of the guanidine function.

Step B8:

The protecting groups, which, as described in Route A, have been used to protect reactive functional groups in amino acid side chains of $AA^1$ and $AA^2$ and in the $R^{2a}R^{2b}N$- component during the coupling steps above, are removed from coupled urea 7 by hydrogenolysis and hydrolysis to give compound 8;

| Compound | Formula |
|---|---|
| 9 | BOC-ACHPA-$NR^{2c}R^{2d}$ |
| 10 | ACHPA-$NR^{2c}R^{2d}$ |
| 11 | BOC-$AA^1$-ACHPA-$NR^{2c}R^{2d}$ |
| 12 | BOC-$AA^2$-$AA^1$-ACHPA-$NR^{2c}R^{2d}$ |
| 13 | $AA^2$-$AA^1$-ACHPA-$NR^{2c}R^{2d}$ |

Peptides of Formula I which contain statine are prepared as described above in routes A and B, except that BOC-ACHPA-OEt, is replaced with BOC-Sta-OEt, resulting in peptides such as 14:

14 $R^{2a}(R^{2b})N$-CO-$AA^2$-$AA^1$-Sta-$NR^{2c}R^{2d}$

Peptides of Formula I which contain amino-ACHPA (AmACHPA) or amino-statine (AmSta) are prepared as described above in Routes A and B, except that BOC-ACHPA-OEt is replaced with BOC-AmACHPA-(CBZ)-OEt or with BOC-AmSta(CBZ)-OEt, with the CBZ group of the AmACHPA or AmSta element being removed by hydrogenolysis in step A9 or B8. In this way, peptides such as 15 (and the corresponding AmSta analog) may be prepared.

15 $R^{2a}(R^{2b})N$-CO-$AA^2$-$AA^1$-AmACHPA-$NR^{2c}R^{2d}$

Peptides of Formula I which contain a 2-substituted statine or ACHPA element as a peptide bond mimic may be prepared as described above in Routes A and B, except that BOC-ACHPA-OEt is replaced with a suitably-protected 2-substituted ACHPA or 2-substituted statine derivative, for example BOC-(2-allyl)-ACHPA-OEt or BOC-(2-isobutyl)Sta-OEt, resulting in peptides such as 16.

16 $R^{2a}(R^{2b})N$-CO-$AA^2$-$AA^1$-(2-isobutyl)ACHPA-$NR^{2c}R^{2d}$

Peptides of Formula I which contain Cal[CH(OH)$CH_2$]Val as a peptide bond mimic are prepared as described below in Routes C and D.

Route C:

Step C1:

The BOC protecting group is removed from lactone 17 by treatment with anhydrous TFA, to give lactone 18;

Step C2:

Lactone 18 is coupled with a BOC-protected amino acid (BOC-$AA^1$) to give 19, or with an N-terminal BOC-protected dipeptide (BOC-$AA^2$-$AA^1$) to give 20, or compound 20 may be prepared by treating 19 with anhydrous TFA, and coupling the product with BOC-$AA^2$ Compound 20 is then treated with anhydrous TFA and the resulting product is treated first with carbonyldiimidazole, and shortly thereafter, with an amino component $R^{2a}R^{2b}NH$, to produce urea 21 or alternatively, the amino component $R^{2a}R^{2b}NH$ is first treated with carbonyldiimidazole or with phosgene, and the resulting intermediate is allowed to react with the product from TFA treatment (above), to produce urea 21;

Step C2a:

Alternatively, compound 19 is treated with anhydrous TFA and the resulting intermediate is coupled to $R^{2a}R^{2b}NCO$-$AA^2$, as described above in Step A5a, providing urea 21;

17 BOC-Cal[CH(OH)$CH_2$]Val lactone
18 Cal[CH(OH)$CH_2$]Val lactone
19 BOC-$AA^1$-Cal[CH(OH)$CH_2$]Val lactone
20 BOC-$AA^2$-$AA^1$-Cal[CH(OH)$CH_2$]Val lactone
21 $R^{2a}(R^{2b})$N-CO-$AA^2$-$AA^1$-Cal[CH(OH)$CH_2$]Val lactone

Step C3:

Lactone 21 is treated with aqueous potassium hydroxide to give the corresponding hydroxyacid, which is treated with t-butyldimethylsilyl chloride and imidazole, then with HOAc in THF-$H_2O$, to give the protected hydroxyacid 22;

22 $R^{2a}(R^{2b})$N-CO-$AA^2$-$AA^1$-Cal[CH(OTBDMS)$CH_2$]Val-OH

Step C4:

Compound 22 is coupled with an amino component $R^{2c}R^{2d}NH$, to give 23, with additional reactive functional groups in the amino component $R^{2c}R^{2d}NH$ being protected with groups such as CBZ for amino and guanidino groups, benzyl ethers for alcohols, and benzyl esters for carboxylic acids;

23 $R^{2a}(R^{2b})$N-CO-$AA^2$-$AA^1$-Cal[CH(OTBDMS)$CH_2$]Val-$NR^{2c}R^{2d}$

Step C5:

The silyl protecting group is removed from 23a (23b) by treatment with tetrabutylammonium fluoride in THF-DMF, and other protecting groups are removed by hydrogenolysis, to give 24;

24(25) $R^{2a}(R^{2b})$N-CO-$AA^2$-$AA^1$-Cal[CH(OH)$CH_2$]Val-$NR^{2c}R^{2d}$

Step C6:

It is understood that during the steps above, the reactive functional groups in the side chains of the amino acids $AA^1$ and $AA^2$ are protected with protecting groups, such as CBZ for amines and guanidines, and benzyl ethers for alcohols, which are removed during the hydrogenolysis of step C5, or when histidine is used as the $AA^1$ component, the side-chain imidazole ring may be protected during the coupling of BOC-$AA^1$ or of BOC-$AA^2$-$AA^1$ to ACHPA of step C2 with a BOC protecting group, which is removed during subsequent treatment with TFA, and the imidazole ring is left unprotected during subsequent N-terminal coupling or acylation reactions, or alternatively, histidine may be protected with a DNP protecting group during step C2, and this DNP group is removed in step C3 during the potassium hydroxide treatment;

Step C7:

In some cases, a quaternized amino group is present in the C-terminal $R^{2c}R^{2d}N$-element of compound 24, and when it is, a tertiary amine within $R^{2c}$ or $R^{2d}$ of $R^{2c}R^{2d}NH$ is quaternized by treatment of $R^{2c}R^{2d}N$-BOC with an alkyl halide and $KHCO_3$ in methanol or ethanol or with an alkyl halide in DMF, with the resulting quaternary ammonium salt being treated with anhydrous TFA to remove the BOC protecting group, resulting in an amine used in coupling step C4 above.

Alternatively, a compound 24 containing a tertiary amine in the $R^{2c}R^{2d}N$- element may be prepared as described above in steps C1 to C6, with the silyl protecting group being removed from 24 by treatment with fluoride. If histidine is present as the $AA^1$ component, the imidazole ring is then protected by treatment with di-t-butyldicarbonate and $Et_3N$ in methanol or DMF, and the tertiary amine present in the $R^{2c}R^{2d}N$- element is then quaternized by treatment with an alkyl halide in DMF.

Step C8:

In cases where a guanidine group is to be introduced into the $R^{2c}R^{2d}N$- component of an inhibitor of Formula I, compound 24 is prepared as described in Steps C1 through C6 above, with Step C7 being also carried out if desired, then an amine group present in the $R^{2c}R^{2d}N$- component and heretofore protected with a BOC group is liberated by treatment of 24 with anhydrous TFA, and standard methods are used for introduction of the guanidine function;

Step C9:

Protecting groups in the molecule are then removed from 24 by hydrogenolysis, to give 25.

Route D:

Step D1:

Lactone 17 is treated with potassium hydroxide to give the corresponding hydroxyacid 26, which is treated with t-butyldimethylsilyl chloride and imidazole, then with HOAc in THF-$H_2O$, to give the protected hydroxyacid 27;

26 BOC-Cal[$CH(OH)CH_2$]Val-OH
27 BOC-Cal[$CH(OTBDMS)CH_2$]Val-OH

Step D2:

Protected hydroxyacid 27 is coupled with an amino component $R^{2c}R^{2d}NH$, to give 28, with additional reactive functional groups in the amino component $R^{2c}R^{2d}NH$, being protected with groups such as CBZ for amino and guanidino groups, benzyl ethers for alcohols, and benzyl esters for carboxylic acids;

Step D3:

The BOC protecting group is removed from 28 by treatment with anhydrous TFA, to give compound 29;

28 BOC-Cal[CH(OTBDMS)$CH_2$]Val-N$R^{2c}R^{2d}$
29 Cal[CH(OTBDMS)$CH_2$]Val-N$R^{2c}R^{2d}$

Step D4:

Compound 29 is coupled with a BOC-protected amino acid (BOC-$AA^1$) or a dipeptide with an N-terminal BOC protecting group (BOC-$AA^2$-$AA^1$), to give coupled product 30 or 31;

30 BOC-$AA^1$-Cal[CH(OTBDMS)$CH_2$]Val-N$R^{2c}R^{2d}$
31 BOC-$AA^2$-$AA^1$-Cal[CH(OTBDMS)$CH_2$]Val-N$R^{2c}R^{2d}$

Step D5:

The N-terminal BOC protecting group of 30 or 31 is removed by treatment with anhydrous TFA in $CH_2Cl_2$, to give 32 or 33;

32 $AA^1$-Cal[CH(OTBDMS)$CH_2$]Val-N$R^{2c}R^{2d}$
33 $AA^2$-$AA^1$-Cal[CH(OTBDMS)$CH_2$]Val-N$R^{2c}R^{2d}$

Step D6:

Compound 33 is treated first with carbonyldiimidazole and shortly thereafter with an amino component $R^{2a}R^{2b}$NH, producing urea 23, or alternatively, the amino component $R^{2a}R^{2b}$NH is first treated with carbonyldiimidazole or with phosgene, and the resulting intermediate is allowed to react with 33, producing urea 23 with additional reactive functional groups in the $R^{2a}$($R^{2b}$)N-element being protected with protecting groups as described above, or alternatively 32 is coupled with $R^{2a}$($R^{2b}$)NCO-$AA^2$, prepared as described in Step A5a, to give urea 23;

23 $R^{2a}$($R^{2b}$)N-CO-$AA^2$-$AA^1$-Cal[CH(OTBDMS)$CH_2$]Val-N$R^{2c}R^{2d}$

Step D7:

The silyl protecting group is removed from 23 by treatment with fluoride, with other protecting groups being removed by hydrogenolysis, to give 24;

Step D8:

In some cases a quaternized amino group is present in the C-terminal $R^{2c}R^{2d}$N- element of compound 24 with a tertiary amine within $R^{2c}$ or $R^{2d}$ of $R^{2c}R^{2d}$NH being quaternized by treatment of $R^{2c}R^{2d}$N-BOC an alkyl halide and $KHCO_3$ in methanol or ethanol or with an alkyl halide in DMF, and the resulting quaternary ammonium salt is treated with anhydrous TFA to remove the BOC protecting group, resulting in an amine used in coupling step D2 above; or
Alternatively, a compound 24 containing a tertiary amine in the $R^{2c}R^{2d}$N- element may be prepared as described above in steps D1 to D6, with the silyl protecting group being removed from 24 by treatment with fluoride, or if histidine is present as the $AA^1$ component, the imidazole ring is protected next by treatment

with di-t-butyldicarbon ate and $Et_3N$ in methanol or DMF, and the tertiary amine present in the $R^{2c}R^{2d}N$- element is quaternized by treatment with an alkyl halide in DMF;

Step D9:

In cases where a guanidine group is to be introduced into the $R^{2c}R^{2d}N$- component of a compound of Formula I, compound 24 is prepared as described above, including Step D8 if desired, then an amine group present in the $R^{2c}R^{2d}N$- component and heretofore protected with a BOC group is liberated by treatment of 24 with anhydrous TFA, and standard methods are used for introduction of the guanidine function;

Step D10:

It is understoood that during the steps above, the reactive functional groups in the side-chains of amino acid components are protected with protecting groups, such as CBZ for amines and guanidines, and benzyl ethers for alcohols, or if histidine is present as $AA^1$, the side chain imidazole ring is protected with a BOC group which is removed by TFA in step D5, and thereafter, the side chain of histidine is left unprotected. Alternatively, histidine with a DNP protecting group may be used, and the DNP group is removed after step D11 by treatment of 25 with thiophenol in DMF.

Step D11:

Protecting groups are removed from 24 by hydrogenolysis, to give 25.

Peptides of formula I which contain sulfamide moieties at the amino-terminus are in general prepared as described above in Routes A to D, except that the sulfamide group is introduced in one of the following ways:

Route E:

Step E1:

A sulfamoyl chloride is prepared using standard procedures, such as by treating an amino component, $R^{2a}R^{2b}NH$, with a ten-fold excess of sulfuryl chloride in anhydrous methylene chloride containing one equivalent of an amine base, such as triethylamine, to give the sulfamoyl chloride, $R^{2a}R^{2b}NSO_2Cl$;

Step E2:

Treatment of this sulfamoyl chloride with an amino acid ester, preferably an amino acid t-butyl ester (for example, phenylalanine t-butyl ester) in the presence of an amine base, such as triethylamine, provides the sulfamate 34;

34 $R^{2a}R^{2b}NSO_2$-NH-Phe-O-t-butyl

Step E3:

Treatment of this sulfamate with anhydrous TFA then affords the sulfamate $R^{2a}R^{2b}NSO_2$-$AA^2$ which is used in Steps A5a, B5a, C2a, or D6, above, in place of $R^{2a}R^{2b}NCO$-$AA^2$;

Step E3a:

Alternatively, the sulfamoyl chloride may be used in the routes described above as an acylating agent, for example, to prepare 35 from 13.

35 $R^{2a}R^{2b}NSO_2$-$AA^2$-$AA^1$-ACHPA-$NR^{2c}R^{2d}$

Route F:

Step F1:

In some case, an unsymmetrical sulfamide is prepared by heating the amino component, $R^{2a}R^{2b}NH$, with sulfamide ($NH_2SO_2NH_2$), affording the monosubstituted sulfamide 36;

36 $R^{2a}R^{2b}NSO_2NH_2$

Step F2:

This sulfamide is then warmed in the presence of an amino acid t-butyl ester (for example, phenylalanine t-butyl ester) to provide the sulfamide 37;

37 $R^{2a}R^{2b}NSO^2$-$AA^2$-O-t-Bu

Step F3:

Treatment of sulfamide 37 with anhydrous TFA then provides the product $R^{2a}R^{2b}NSO_2$-$AA^2$ (for example, 38) which is used as described in Step E3, above.

38 $R^{2a}R^{2b}NSO_2NH$-Phe

Other references pertinent to the synthesis of sulfamides include: S.D. McDermott et al, Organic Preparations and Procedures Int., 1984, 16, 49 77 and W.T. Comer et al, U.S. Patent 4,044,151 (1977).

The novel peptides of the present invention possess a high degree of activity in treating renin-associated hypertension, hyperaldosteronism and/or congestive heart failure in humans, as well as in other warm-blooded animals such as mice, rats, horses, dogs and cats.

For these purposes, the peptides of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically acceptable carriers, adjuvants and vehicles.

Thus, in accordance with the present invention there is further provided a method of treating and a pharmaceutical composition for treating renin-associated hypertension, hyperaldo steronism, and/or congestive heart failure. This treatment involves administering to a patient in need of such treatment a pharmaceutical composition comprising a pharmaceutical carrier and a therapeutically-effective amount of a peptide of the formula:

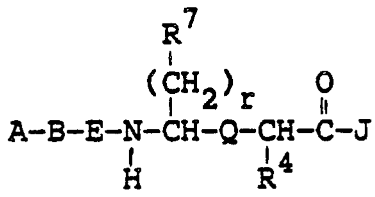

(I) ,

wherein A, B, $R^{1a}$, $R^1$, $R^7$, Q, $R^4$, r and J are defined above, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions; or suppositories.

When administered orally as a suspension, these compositions may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquify and/or dissolve in the rectal cavity to release the drug.

Dosage levels of the order of 0.02 to 2.0 grams-per-day are useful in the treatment of the above-indicated conditions, with oral doses two to-five times higher. For example, renin-associated hypertension and hyperaldosteronism are effectively treated by the administration of from 10 to 50 milligrams of the compound per kilogram of body weight from one to three times per day. It will be understood, however, that the specific dose level and frequency of dosage for any particular patient will depend upon a variety of factors including the activity of the specific compound employed, the metabolic stability and length of action of that compound, the age, body weight, general health, sex, diet, mode and time of administration, rate of excretion, drug combination the severity of the particular condition, and the host undergoing therapy.

The present invention is also directed to combinations of the novel renin-inhibitory peptides of Formula I with one or more antihypertensive agents selected from the group consisting of diuretics, α-and/or β-adrenergic blocking agents, CNS-acting agents, adrenergic neuron blocking agents, vasodilators, angiotensin I converting enzyme inhibitors, calcium channel blockers, and other antihypertensive agents.

For example, the compounds of this invention can be given in combination with such compounds or salt or other derivative forms thereof as:

Diuretics:

acetazolamide; amiloride; bendroflumethiazide; benzthiazide; bumetanide; chlorothiazide; chlorthalidone; cyclothiazide; ethacrynic acid; furosemide; hydrochlorothiazide; hydro flumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents:

dibenamine; phentolamine; phenoxybenzamine; prazosin; tolazoline;

β-Adrenergic Blocking Agents:

atenolol; metoprolol; nadolol; propranolol, timolol;
((±)-2-[3-(tert-butylamino)-2-hydroxypropoxy]-2-furananilide) (ancarolol);
(2-acetyl-7-(2-hydroxy-3-isopropylaminopropoxy)benzofuran HCl) (befunolol);
((±)-1-(isopropylamino)-3-(p-(2-cyclopropylmethoxyethyl)-phenoxy)-2-propranol HCl) (betaxolol);
(1-[(3,4-dimethoxyphenethyl)amino]-3-(m-tolyloxy)-2-propanol HCl) (bevantolol);
((±)-1-(4 -(2-isopropoxyethoxy)methyl)phenoxy)-3-isopropylamino-2-propanol)fumarate) (bisoprolol);
(4-(2-hydroxy-3-[4-(phenoxymethyl)-piperidino]-propoxy)-indole);
(carbazolyl-4-oxy-5,2-(2-methoxyphenoxy)-ethylamino-2-propanol);
(1-((1,1-dimethylethyl)amino)-3-((2 methyl-1H-indol-4-yl)oxy)-2-propanol benzoate) (bopindolol);
(1-(2-exobicyclo[2.2.1]-hept-2-ylphenoxy)-3-[(1-methylethyl)-amino]-2-propanol HCl) (bornaprolol);
(o-[2-hydroxy-3-[(2-indol-3-yl-1,1-dimethylethyl)amino]propoxy]benzonitrile HCl) (bucindolol);
(α-[(tert.butylamino)methyl]-7-ethyl-2-benzofuranmethanol) (bufuralol);
(3-[3-acetyl-4 [3-(tert.butylamino)-2-hydroxypropyl]-phenyl]-1,1-diethylurea HCl) (celiprolol);
((±)-2-[2-[3-[(1,1-dimethylethyl)amino]-2-hydroxypropoxy]phenoxy]-N-methylacetamide HCl) (cetamolol);
(2-benzimidazolyl-phenyl(2-isopropylaminopropanol));
((±)-3′-acetyl-4′-(2-hydroxy-3-isopropylaminopropoxy)acetanilide HCl) (diacetolol);
(methyl-4-[2-hydroxy-3-[(1-methylethyl)aminopropoxy]]-benzenepropanoate HCl) (esmolol);
(erythro-DL-1-(7-methylindan-4-yloxy)-3-isopropylaminobutan-2-ol);
(1-(tert.butylamino)-3-[O-(2-propynyloxy)phenoxy]-2-propanol (pargolol);
(1-(tert.butylamino)-3-[o-(6-hydrazino-3-pyridazinyl)phenoxy]-2-propanol diHCl) (prizidilol);
((-)-2-hydroxy-5-[(R)-1-hydroxy-2-[(R)-(1 methyl-3-phenylpropyl)amino]ethyl]benzamide);
(4-hydroxy-9-[2-hydroxy-3-(isopropylamino-propoxy]-7-methyl-5H-furo[3,2-g][1]-benzopyran-5-one) (iprocrolol);
((-)-5-(tert.butylamino)-2-hydroxypropyl]-3,4-dihydro-1-(2H)-naphthalenone HCl) (levobunolol);
(4-(2-hydroxy-3-isopropylamino-propoxy)-1,2-benzisothiazole HCl);
(4-[3-(tert.butylamino)-2-hydroxypropoxy]-N-methylisocarbostyril HCl);
((±)-N-2-[4-(2-hydroxy-3-isopropyl aminopropoxy)phenyl]ethyl-N′-isopropylurea) (pafenolol);
(3-[[(2-trifluoroacetamido)ethyl]amino]-1-phenoxypropan-2-ol);
(N-(3-(o-chlorophenoxy)-2-hydroxypropyl)-N-(4′-chloro-2,3-dihydro-3-oxo-5-pyridazinyl)ethylenediamine);
((±)-N-[3-acetyl-4-[2-hydroxy-3-[(1-methylethyl)amino]propoxy]phenyl]butanamide) (acebutolol);
((±)-4′-[3-(tert-butylamino)-2-hydroxypropoxy]spiro[cyclohexane-1,2′-indan]-1′-one)-(spirendolol);
(7-[3-[[2 hydroxy-3-[(2-methylindol-4-yl)oxy]propyl]amino]butyl]thiophylline) (teoprolol);
((±)-1-tert.butylamino-3-(thiochroman-8-yloxy)-2-propanol) (tertatolol);
((±)-1-tert.butylamino-3-(2,3-xylyloxy)-2-propanol HCl) (xibenolol);
(8-[3-(tert.butylamino)-2-hydroxypropyl]-5-methylcoumarin) (bucumolol);
(2-(3-(tert.butylamino)-2-hydroxy-propoxy)benzonitrile HCl) (bunitrolol);
((±)-2′-[3-(tert-butylamino)-2-hydroxypropoxy-5′-fluorobutyrophenone) (butofilolol);
(1-(carbazol-4-yloxy)-3-(isopropylamino)-2-propanol) (carazolol);
(5-(3-tert.butylamino-2-hydroxy)propoxy-3,4-dihydrocarbostyril HCl) (carteolol);
(1-(tert.butylamino)-3-(2,5-dichlorophenoxy)-2-propanol) (cloranolol);
(1-(inden-4(or-7)-yloxy)-3-(isopropylamino)-2-propanol HCl) (indenolol);
(1-isopropylamino-3-[(2-methylindol-4-yl)oxy]-2-propanol) (mepindolol);
(1-(4-acetoxy-2,3,5-trimethylphenoxy)-3-isopropylaminopropan-2-ol) (metipranolol);
(1-(isopropylamino)-3-(o-methoxyphenoxy)-3-[(1-methylethyl)amino]-2-propanol) (moprolol);
((1-tert.butylamino)-3-[(5,6,7,8 tetrahydro-cis-6,7-dihydroxy-1-naphthyl)oxy]-2-propanol) (nadolol);
((S)-1-(2-cyclopentylphenoxy)-3-[(1,1-dimethylethyl)amino]-2-propanol sulfate (2:1)) (penbutolol);
(4′-[1-hydroxy-2-amino)ethyl]methanesulfonanilide) (sotalol);
(2-methyl-3-[4-(2-hydroxy-3-tert.butylaminopropoxy)phenyl]-7-methoxy-isoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxypropoxy]-β-methylcinnamonitrile) (pacrinolol);
((±)-2-(3′-tert.butylamino-2′-hydroxypropylthio)-4-(5′-carbamoyl-2-thienyl)thiazole HCl) (arotinolol);
((±)-1-[p-[2-(cyclopropylmethoxy)ethoxy]phenoxy]-3-(isopropylamino)-2-propanol) (cicloprolol);
((±)-1-[(3-chloro-2-methylindol-4-yl)oxy]-3-[(2-phenoxyethyl)amino]-2-propanol) (indopanolol);
((±)-6-[[2-[[3-(p-butoxyphenoxy)-2-hydroxypropyl]amino]ethyl]amino]-1,3-dimethyluracil) (pirepolol);
(4-(cyclohexylamino)-1-(1-naphtholenyloxy)-2-butanol);
(1-phenyl-3-[2-[3-(2-cyanophenoxy)-2-hydroxypropyl]aminoethyl]hydantoin HCl);

(3,4-dihydro-8-(2-hydroxy-3-isopropylaminopropoxy)-3-nitroxy-2H-1-benzopyran) (nipradolol);

α- and β-Adrenergic Blocking Agents:

((±)-1-tert-butylamino)-3-[o-[2-(3-methyl-5-isoxazolyl)vinyl]phenoxy]-2-propanol) (isoxaprolol);
(1-isopropylamino-3-(4-(2-nitroxyethoxy)phenoxy)-2-propanol HCl);
(4 hydroxy-α-[[3-(4-methoxyphenyl)-1-methylpropyl]aminomethyl]-3-(methylsulfinyl)-benzmethanol HCl) (sulfinalol);
(5-[1-hydroxy-2-[[2-(o-methoxyphenoxy)ethyl]amino]ethyl]-2-methylbenzenesulfonamide HCl);
(5-[1-hydroxy-2-[(1-methyl-3-phenylpropyl)amino]ethyl]salicylamide HCl) (labetalol);
(1-((3-chloro-2-methyl-1H-indol-4-yl)oxy)-3-((2-phenoxyethyl)amino)-2-propanol-hydrogenmalonate) (ifendolol);
(4-(2-hydroxy-3-[(1-methyl-3-phenylpropyl)amino]propoxy)benzeneacetamide);
(1-[3-[[3-(1-naphthoxy)-2-hydroxypropyl]-amino]-3,3-dimethyl-propyl]-2-benzimidazolinone);
(3-(1-(2-hydroxy-2-(4-chlorophenylethyl)-4-piperidyl)-3,4-dihydroxy)quinoxolin-2(1H)-one);

CNS-Acting Agents:

clonidine; methyldopa;

Adrenergic Neuron Blocking Agents:

guanethidine; reserpine and other rauwolfia alkaloids such as rescinnamine;

Vasodilators:

diazoxide; hydralazine; minoxidil;

Angiotensin I Converting Enzyme Inhibitors:

1-(3-mercapto-2-methyl-1-oxopropyl)-L-proline (captopril);
(1-(4-ethoxycarbonyl-2,4(R,R)-dimethylbutanoyl)indoline-2(S)-carboxylic acid);
(2-[2-[[1-(ethoxycarbonyl)-3-phenyl-propyl]amino]-1-oxopropyl]-1,2,3,4-tetrahydro-3-isoquinoline carboxylic acid);
((S)-1-[2-[[1-ethoxycarbonyl)-3-phenylpropyl]amino]-1-oxopropyl]octahydro-1H-indole-2-carboxylic acid HCl);
(N-cyclopentyl-N-)3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)cis,syn-octahydroindol-2(S)-carboxylic acid HCl);
((-)-(S)-1-[(S)-3-mercapto-2-methyl-1-oxopropyl]indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino)-2,3,4,5-tetrahydro-2-oxo-1-(3S)-benzazepine-1-acetic acid HCl);
(N-(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
$N^2$-[1-(S)-carboxy-3-phenylpropyl]-L-lysyl-L-proline (lisinopril);

Calcium Channel Blockers:

α-[3-[[2-(3,4-dimethoxyphenyl)ethyl]methylamino]propyl]-3,4-dimethoxy-α-(1-methylethyl)benzeneacetonitrile (verapamil);
1,4-dihydro-2,6-dimethyl-4-(2-nitrophenyl)-3,5-pyridinedicarboxylic acid dimethyl ester (nifedipine);

2-(2,2-dicyclohexylethyl)piperidine (perhexiline);
N-(1-methyl-2-phenylethyl)-phenylbenzenepropanamine (prenylamine);
3-(aminosulfonyl)-4-chloro-N-(2,3-dihydro-2-methyl-1H-indol-1-yl)benzamide (indapamide);
(2′-(2 diethylaminoethoxy)-3-phenylpropiophenone (etafenone);
(4-[4,4-bis-(4-fluorophenyl)butyl]-N-(2,6-dimethylphenyl)-1-piperazineacetamide)-(lidoflazine);
(2-(N-benzyl-N-methylamino)ethylmethyl-2,6-dimethyl-4-(m-nitrophenyl)-1,4-dihydro-3,5-pyridinedicarboxylate HCl) (nicardipine);
(N-(3,4-dimethoxyphenethyl)-2-(3,4-dimethoxyphenyl)-N-methyl-m-dithiane-2-propylamine-1,1,3,3-tetraoxide) (tiapamil);
(5,6-dimethoxy-2-(3-[(α-(3,4-dimethoxy)phenylethyl)methylamino]propyl)phthalimidine) (falipamil);
(β-[(2-methylpropoxy)methyl]-N-phenyl-N-phenylmethyl-1-pyrrolidineethanamine HCl monohydrate) (bepridil);
((+)-cis-3-(acetyloxy)-5-[2-(dimethylamino)ethyl]-2,3-dihydro-2-(4-methoxyphenyl)-1,5-benzothiazepin-4-(5H)-one) (diltiazem);
((E)-1-[bis-(p-fluorophenyl)methyl]-4-cinnamylpiperazine di HCl) (flunarizine);
(5-[(3,4-dimethoxyphenethyl)methylamino]-2-isopropyl-2-(3,4,5-trimethoxyphenyl)valeronitrile (gallopamil);
(ethylmethyl(2,3-dichlorophenyl)-1,4-dihydro-2,6-dimethyl-3,5-pyridinedicarboxylate (felodipine);
(isopropyl-2-methoxyethyl-1,4-dihydro-2,6-dimethyl-4-(3-nitrophenyl)-3,5-pyridinecarboxylate) (nimodipine);
(3-ethyl-5-methyl-1,4-dihydro-2,6-dimethyl-4-(3-nitrophenyl)-3,5-pyridine-dicarboxylate) (nitrendipine);

Other Antihypertensive Agents:

aminophylline; cryptenamine acetates and tannates; deserpidine; meremethoxylline procaine; pargyline; trimethaphan camsylate; and the like, as well as admixtures and combinations thereof.

Typically, the individual daily dosages for these combinations can range from about one-fifth of the minimally-recommended clinical dosages to the maximum recommended levels for the entities when they are given alone. Coadministration is most readily accomplished by combining the active ingredients into a suitable unit dosage form containing the proper dosages of each. Other methods of coadministration are, of course, possible.

The renin-inhibitory novel peptides of the present invention may also be utilized in in vivo or in vitro diagnostic methods for the purpose of establishing the significance of renin as a causative or contributory factor in hypertension, hyperaldosteronism or congestive heart failure in a particular patient.

In the in vivo method, a novel peptide of the present invention is administered to a patient, preferably by intravenous injection, although parenteral administration is also suitable, at a hypotensive dosage level in a single dose of from 0.1 to 10 mg per kg of body weight, and the resulting transitory fall in blood pressure, if it occurs, indicates supranormal plasma renin levels.

In vitro methods which may be employed involve incubating a body fluid, preferably plasma, with a novel peptide of the present invention according to methods described in Boger et al., J. Med. Chem., 1985, 28, 1789-1790.

Pepstatin may be employed in the methods described above as an active control (see, e.g., U.S. Patent Nos. 3,784,686 and 3,873,681 for a description of the use of pepstatin in diagnostic methods of this type).

The following examples are intended to be representative and not limiting. $IC_{50}$ results were obtained according to the methods described in J. Med. Chem., 1985, 28, 1789-1790.

## EXAMPLE 1

### Preparation of N-(Morpholin-4-yl)carbonyl-Phenylalanine

To a suspension of phenylalanine methyl ester hydrochloride (1 gm, 0.00465 mol) in methylene chloride (10 ml) was added triethylamine (2 ml) followed by carbonyldiimidazole (0.83 gm, 1 equiv.). After stirring for 1 hr at room temperature, morpholine (0.8 ml, 2 equiv.) was added and the reaction was stirred a further 2 hr until TLC (silica, EtOAc) showed the reaction to be complete. The reaction mixture was washed once with HCl (IN), then dried over $Na_2SO_4$, filtered, evaporated in vacuo, and purified by chromatography (silica,

EtOAc), to give N-(morpholin-4-yl)carbonylphenylalanine methyl ester (1 gm). TLC (silica, EtOAc) Rf= 0.52. NMR ($CDCl_3$) 3.1(m,2H), 3.25-3.35(m,4H), 3.65-3.7(m,4H), 3.75(s,3H), 4.75.85(m,lH), 7.1(d,2H), 7.3(m,3H).

To a solution of this material (0.485 gm) in methanol (3 ml) at room temperature, a solution of KOH (1N, 5.3 ml) was added and the mixture was stirred for 3 hr. The reaction mixture was acidified with HCl (3N), and extracted with methylene chloride, then the combined organic fractions were dried over $NaSO_4$, filtered and evaporated to dryness to give 0.39 gm of the title compound. NMR ($CDCl_3$) 3.2(m,2H), 3.25-3.35-(m,4H), 3.35-3.45(m,4H), 4.75-4.86(m,1H), 7.2(d,2H), 7.25-7.35(m,3H).

## EXAMPLE 2

### Preparation of N-(4-Boc-Piperazinyl)Carbonyl-Phenylalanine

N-(4-Boc-piperazinyl)carbonyl-phenylalanine was prepared according to the procedures of Example 1, using N-Boc-piperazine in place of morpholine. NMR($CDCl_3$) 1.45(s,9H), 3.1-3.2(2d,2H), 3.3-3.4(m,4H), 3.3-3.45(m,4H), 4.55-4.65(m,1H), 4.8(d,1H), 7.2(d,2H), 7.25-7.35(m,3H).

## EXAMPLE 3

### Preparation of Boc-(im-DNP)-Histidyl-ACHPA-2(S)-Methylbutylamide

Boc-ACHPA-2(S)-methylbutylamide (0.85 gm, 0.0022 mol) was dissolved in a saturated solution of HCl in methanol and the reaction was stirred for 1 hr, at which time it was thoroughly evaporated in vacuo. To the amine hydrochloride in methylene chloride (10 ml) at 0° C was added triethylamine (1 ml, 3 equiv.), and after 15 min, Boc-N-im-DNP-histidine (1 gm, 1.1 equiv.), then 1-hydroxybenzotriazole hydrate (0.326 gm) and dicyclohexylcarbodiimide (0.5 gm).

The reaction mixture was slowly warmed to room temperature and stirred until reaction was complete (12 hr). The mixture was filtered to remove dicyclohexylurea and the filtrate was successively washed with 1N HCl (twice), and sat. $NaHCO_3$(twice), then dried over $Na_2SO_4$, filtered and evaporated in vacuo.

The product was purified (Chromatography, silica, EtOAC) to give the title compound (1 gm). TLC (silica, EtOAC) Rf=0.68. NMR ($CDCl_3$) 0.85 0.95(m,6H), 1.0 1.3(m,6H), 1.3-1.7 (m,9H), 1.4(s,9), 1.7-1.8-(d,lH), 2.18-2.25(dd,1H), 2.35-2.45(m,1H), 3.0-3.2(m,4H), 3.85-4.0(2H), 4.35-4.45(ABq,1H), 6.2(brd,1H), 6.48-(brt,1H), 6.55(d,1H), 6.95(s,1H), 7.65(s,1H), 7.75(d,1H), 8.6(dd,1H), 8.85(dd,1H).

## EXAMPLE 4

### Preparation of N-(Morpholin-4-yl)carbonyl-Phenylalanyl-Histidyl-ACHPA-2(S)-Methylbutylamide

A. Morpholin-4-yl)carbonyl-Phenylalanyl-(N-imDNP)-Histidyl-ACHPA-2(S)Methylbutyl Amide

Boc-(N-im-DNP)histidyl-ACHPA-2(S)methylbutyl amide (0.4 gm, 0.00058 mol) in a saturated solution of HCl in MeOH was stirred one hr at room temperature, whereupon the solution was thoroughly evaporated in vacuo. This product was dissolved in methylene chloride (5ml) and mixed with diisopropylethylamine (0.4 ml), and N-(morpholin-4-yl)carbonylphenylalanine (0.15 gm), then benzotriazol-1-yloxytris(dimethylamino)-phosphonium-hexafluorophosphate ("BOP") (0.26 gm, 1.2 equiv.) was added. The reaction mixture was stirred 12 hr at room temperature, whereupon it was evaporated in vacuo, redissolved in EtOAC, and washed with HCl (1N) and sat. $NaHCO_3$. The resulting EtOAc solution was dried over $Na_2SO_4$, filtered and evaporated in vacuo, and this product was chromatographed (silica, 9:1:0.5 EtOAc:Acetonitrile:MeOH) to give the title compound.

B. N-(Morpholin-4-yl)carbonyl-Phenylalanyl-Histidyl-ACHPA-2-(S)Methylbutyl Amide

Thiophenol (0.05 ml) was added to a solution of the product of Step A in methanol (1 ml), and after stirring for 1 hr, the reaction mixture was thoroughly evaporated. The product was chromato graphed (LH-20, MeOH) to give the title compound (0.045 gm). HPLC (RP-18,8:2 MeOH:$H_2O$) rt= 7.22 min. MS, m/e=682(M+1), NMR:consistent with assigned structure. $IC_{50}$ =5nM.

EXAMPLE 5

Preparation of N-(4-Boc-Piperizinyl)Carbonyl-Phenylalanyl-Histidyl-ACHPA-2(S)Methylbutylamide

This compound was prepared according to Example 4 using N-(4-Boc-piperizinyl)carbonylphenylalanine prepared in Example 2. TLC (silica, 85:15 EtOAC:MeOH) Rf=0.37. MS, m/e=781(M+1). NMR:consistent with assigned structure. $IC_{50}$ =10nM.

EXAMPLE 6

N-(Piperazin-1-yl)carbonyl-Phenylalanyl-Histidyl-ACHPA-2(S)-Methylbutylamide

N-(piperazin-1-yl)carbonyl-phenylalanylhistidyl-ACHPA-2(S)methylbutylamide was prepared from N-(4-Boc-piperazinyl)carbonyl-phenylalanyl-histidyl-ACHPA-2(S)-methylbutylamide from Example 5 by stirring in a solution of MeOH saturated with HCl for 30 min, then evaporating to dryness in vacuo. HPLC (RP-18, 75:25 MeOH:$H_2O$) rt=15.78 min. MS, m/e=681(M+1). NMR:consistent with assigned structure. $IC_{50}$ =43nM.

EXAMPLE 7

Preparation of N-(morpholin-4-yl)carbonyl-L-Phenylalanyl-L-Histidyl(3S,4S)-4-Amino-5-Cyclohexyl-3-Hydroxypentanoyl-1-Methylquinuclidinium-3-yl-Amide Acetate

To 10 ml dry, degassed DMF was added N-(morpholin-4-yl)carbonyl-L phenylalanine (925 mg, 3.47 indole), 1-(3-dimethylaminopropyl)-3-ethylcarbodiimide HCl(EDC) (665 mg, 3.47 mmole) and N-hydroxybenzotriazole • $H_2O$ (532 mg, 3.47 mmole). L-Histidyl(DNP)-(3S,4S)-4-amino-5-cyclohexyl-3 hydroxypentanoic ethyl ester trifluoroacetate (2.29 g, 3.47 mmole) and triethylamine (1.45 ml, 10.41 mmole) were added to the reaction mixture and the resulting mixture was stirred under ntirogen for 3 days. 2 ml of 50% acetic acid was added and the mixture was rotevaporated to an oil, which was dissolved in ethyl acetate and washed with water (4x). Removal of the EtOAc afforded 2.76 g crude product which was then DNP-deprotected using 10% thiophenol in DMF (4 hours r.t.) to give 981 mg of N-(morpholin-4-yl)carbonyl-L phenylalanyl-L-histidyl-(3S,4S)-4 amino-5-cyclohexyl-3-hydroxypentanoic acid ethyl ester after chromatography ($CHCl_3$:MeOH:$H_2O$, 100:10:1; 5 cm x 60 cm).

Conversion of the ethyl ester to hydrazide and azide coupling (0.57 mmole) to 3-aminoquinuclidine were carried out according to the standard methods. Chromatographic purification of the crude amide afforded N-(morpholin-4-yl)carbonyl-L-Phenylalanyl-L-Histidyl-(3S,4S)-4-amino-5-cyclohexyl-3-hydroxy pentanoyl-3-quinuclidinyl amide (153 mg). NMR (360 Mhz) and FAB mass spectral analysis (M+H=721 m/e) were consistent with the proposed structure. $IC_{50}$ =30nM.

Quaternization of this amide (125 mg, 158 µmoles) using methyl iodide (10.3 µl, 165 µmoles) in 300 µl $CHCl_3$ proceeded cleanly to the desired salt overnight. The residue after rotevaporation was dissolved in dilute acetic acid, passed through BioRad AG3 X4A (500 mg) and concentrated to dryness prior to chromatography of quaternary salt using 60:40:3:6 $CHCl_3$:MeOH:$H_2O$: $NH_3$, 230-400 MeOH Whatman G60

silica gel, 2 cm x 25 cm. The product fractions were freeze-dried from dioxane to afford 110 mg of the title compound. NMR (360 Mhz) and FAB mass spectral analysis ($M^+ = 735$ m/e) were consistent with the proposed structure. $IC_{50} = 29nM$.

## Claims

1. A peptide of the formula:
A-B-E-G-J,
wherein:
A is
hydrogen;

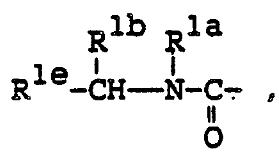

where
$R^{1a}$ and $R^{1b}$ are independently hydrogen or $C_1$-$C_4$-alkyl or $R^{1a}$ and $R^{1b}$ taken together are $-(CH_2)_3-$;
$R^{1e}$ is hydrogen; aryl, wherein aryl is unsbustituted or mono-, di- or trisubstituted phenyl or naphthyl, where the substituent(s) is/are independently selected from the group consisting of $C_1$-$C_8$-alkyl, amino, mono- or di-$C_1$-$C_4$-alkylamino, amino-$C_1$-$C_4$-alkyl, hydroxy-$C_1$-$C_4$-alkyl, phenyl-$C_1$-$C_4$-alkyl, mono-or di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2$-$C_1$-$C_4$-alkyl, $CO_2$-$C_1$-$C_4$-alkoxy, $NR^5R^6$, and $N(R^5)_3^{\oplus} A^{\ominus}$, wherein $R^5$ and $R^6$ are independently hydrogen, unsubstituted or monosubstituted $C_1$-$C_4$-alkyl, where the substituent is amino, mono- or di-$C_1$-$C_4$-alkylamino, hydroxyl, $C_1$-$C_4$-alkoxy or $N(C_1\text{-}C_4\text{alkyl})_3^{\oplus} A^{\ominus}$; and $A^{\ominus}$ is a counterion selected from the group consisting of small, single negatively-charged ions, such as chloride, bromide, nitrate, perchloride, benzoate, maleate, benzene sulfonate, tartrate, hemitartrate and acetate; Het, wherein Het is an unsubstituted or mono- or disubstituted 5- to 7-membered mono- or bicyclic or 7- to 10-membered bicyclic heterocyclic ring, where the one or two heteroatoms are independently selected from the group consisting of N, O, S, NO, SO, $SO_2$ and quaternized N, and the substituent(s) is/are independently selected from the group consisting of hydroxyl, thiol, $C_1$-$C_6$-alkyl, $CF_3$, $C_1$-$C_4$-alkoxy, halo, aryl, as defined above, aryl-$C_1$-$C_4$-alkyl, amino, mono-or di-$C_1$-$C_4$-alkylamino, amino-$C_1$-$C_4$-alkylamino, amino-$C_1$-$C_4$-alkyl, hydroxy-$C_1$-$C_4$-alkyl, di-$C_1$-$C_4$-alkyl-amino-$C_1$-$C_4$-alkyl, guanidyl, guanidyl-$C_1$-$C_4$-alkyl, CHO, $CO_2H$, $CO_2$-$C_1$-$C_4$-alkyl, $NR^5R^6$, and $N(R^5)_3^{\oplus} A^{\ominus}$ wherein $R^5$, $R^6$ and $A^{\ominus}$ are as defined above, or alternatively when N is present as a heteroatom, the substituents are $-(CH_2)_q-$ or $-(CH_2)_2-O-(CH_2)_2$ and form a quaternary spirocyclic ring with the N atom, wherein q is 3-to-6; $-CO_2R^{1a}$ or $-CONR^{1a}R^{1b}$, wherein $R^{1a}$ and $R^{1b}$ are as defined above; $C_3$-$C_8$-cycloalkyl; $-CONHSO_2$-aryl, wherein aryl is as defined above; or unsubstituted or mono- or disubstituted $C_1$-$C_4$-alkyl, wherein the substituent(s) is/are independently selected from the group consisting of $C_3$-$C_7$-cycloalkyl; Het, as defined above; aryl, as defined above; $-CO_2R^{1a}$ or $-CONR^{1a}R^{1b}$, where $R^{1a}$ and $R^{1b}$ are as defined above; $-CONHSO_2$-aryl, where aryl is as defined above; $R^{1c}R^{1d}N-$, where $R^{1c}$ and $R^{1d}$ are independently hydrogen; unsubstituted or monosubstituted $C_1$-$C_4$-alkyl, where the substituent is $CO_2R^{1a}$ or $CONR^{1a}R^{1b}$, wherein $R^{1a}$ and $R^{1b}$ are as defined above; aryl $C_1$-$C_4$-alkyl, wherein aryl is as defined above; $-CONR^5R^6$, where $R^5$ and $R^6$ are as defined above; or unsubstituted or monosubstituted $C_2$-$C_4$-alkyl, wherein the substituent is on the terminal carbon atom and is -OH or $-NR^{1a}R^{1b}$, where $R^{1a}$ and $R^{1b}$ are as defined above; -OH; $-SO_3H$; guanidino; $-CO_2H$; $-CO_2$-$C_1$-$C_4$-alkyl; and $-CO$-$NH$-$SO_2$-aryl;

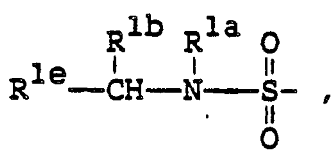

where

$R^{1a}$, $R^{1b}$, $R^{1c}$, $R^{1d}$ and $R^{1e}$ are as defined above;

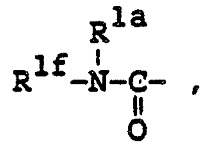

where
$R^{1f}$ is aryl, as defined above, or Het, as defined above; and $R^{1a}$ is as defined above;

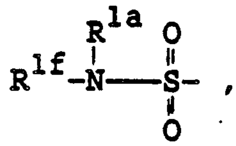

where
$R^{1f}$ and $R^{1a}$ are as defined above;

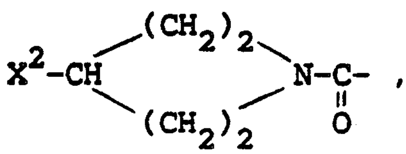

where
$X^2$ is O, S, SO, $SO_2$, N-$R^{1c}$, wherein $R^{1c}$ is as defined above;

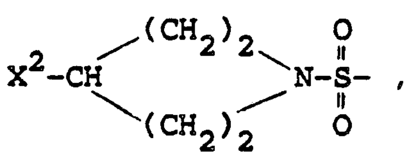

where
$X^2$ is as defined above;

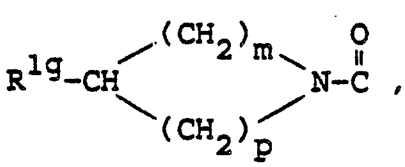

where
m and p are independently 1 or 2; and
$R^{1g}$ is hydroxyl; -$NR^{1c}R^{1d}$, wherein $R^{1c}$ and $R^{1d}$ are as defined above, or any of the definitions of $R^{1e}$, as listed above;

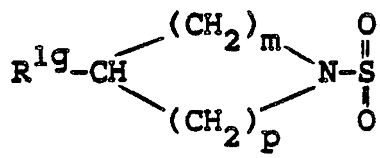 ,

where
$R^{1g}$, m and p are as defined above;
B is

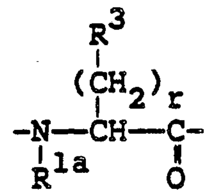 ,

where
r is 1-to-4;
$R^3$ is hydrogen; $C_1$-$C_4$-alkyl; aryl, as defined above; or indolyl; and
$R^{1a}$ is as defined above;

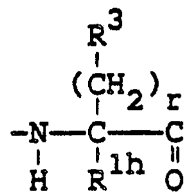

where
$R^{1h}$ is $C_1$-$C_4$-alkyl; and
$R^3$ and r are as defined above; or

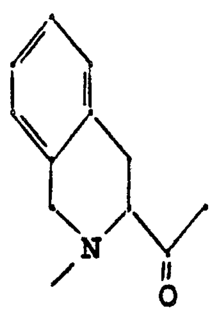 ;

E is

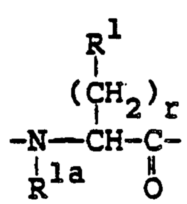 ,

where

r is as defined above; and

$R^1$ is hydrogen; aryl, as defined above; Het, as defined above; or unsubstituted or mono- or disubstituted $C_1$-$C_4$-alkyl, wherein the substitutent(s) is/are on the terminal carbon atom(s) and is/are chosen from the group consisting of -$CO_2R^{1a}$ and -$CONR^{1a}R^{1b}$, where $R^{1a}$ and $R^{1b}$ are as defined above; and when r is 2, 3 or 4, alternatively $C_1$-$C_4$-alkyl, -$NH_2$, and -OH; or

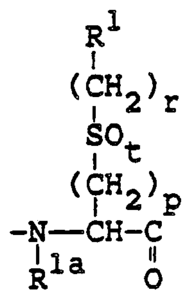
,

where

t is 0-to-2; and

$R^1$, $R^{1a}$, p and r are as defined above;

G is

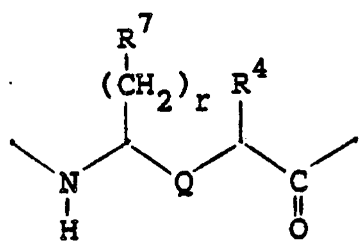
,

where

$R^4$ is hydrogen; $C_1$-$C_8$-alkyl; $C_2$-$C_8$-alkenyl; mono- or disubstituted $C_2$-$C_8$- alkyl, wherein the substituent(s) is/are on the final 1 or/and 2 carbon atoms of the alkyl chain and is/are independently selected from the group consisting of hydroxyl, carboxy, $CO_2$-$C_1$-$C_4$-alkyl, amino, mono-, di-, or tri- $C_1$-$C_4$-alkylamino, guanidyl, $NR^5R^6$ and $N(R^5)_3{}^{\oplus}A^{\ominus}$, wherein $R^5$, $R^6$ and $A^{\ominus}$ are as defined above; aryl, as defined above; unsubstituted or mono-, di-or trisubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy, and halo;

$R_7$ is hydrogen; $C_3$-$C_6$-alkyl; aryl, as defined above; or unsubstituted or mono-, di- or trisubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy, and halo;

Q is

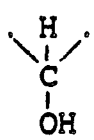 ; 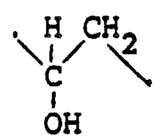 ; 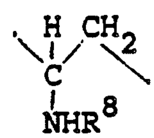 or 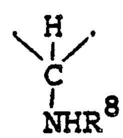 .

wherein

$R^8$ is H; $C_1$-$C_4$-alkyl; $C_1$-$C_4$-alkanoyl; aroyl; $C_1$-$C_4$ alkoxycarbonyl; aryloxycarbonyl; or aryl $C_1$-$C_4$-alkoxycarbonyl, where aryl is as defined above; and

r is as defined above; and

J is -Y-$(CH_2)_n$-$R^{10}$,

where

n is 0-to-5; and

Y is -NH-, -O- or $N(CH_2)_n-R^{10}$, wherein $R^{10}$ is hydrogen; hydroxy; $C_1$-$C_4$-alkyl; $C_3$-$C_7$-cycloalkyl; $C_1$-$C_4$-alkyl-$SO_2OH$; aryl, as defined above; $NR^{11a}R^{11b}$, wherein $R^{11a}$ and $R^{11b}$ are independently hydrogen; unsubstituted or mono-substituted $C_1$-$C_4$-alkyl, wherein the substituent is carboxy, sulfo or hydroxy; monosubstituted $C_2$-$C_4$-alkyl, wherein the substituent is on the terminal carbon and is amino or mono- or di-$C_1$-$C_4$-alkylamino; aryl, as defined above; aryl-$C_1$-$C_4$-alkyl; Het, as defined above; Het-$C_1$-$C_4$-alkyl; $N(R^{11a})\overset{\oplus}{_3}$ $A^{\ominus}$, where $R^{11a}$ and $A^{\ominus}$ are as defined above; guanidino-$C_2$-$C_4$-alkyl; or guanidyl; except that when n is 0 or 1, $R^{10}$ cannot be hydroxy or $NR^{11a}R^{11b}$;

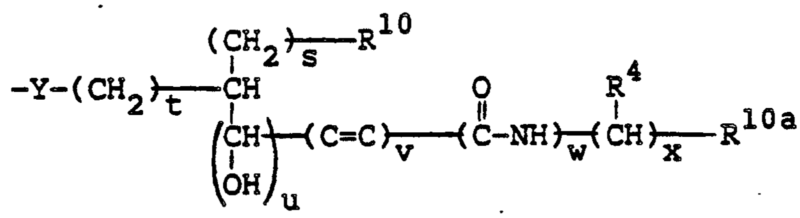

where

$R^{10a}$ is independently one of the definitions of $R^{10}$,

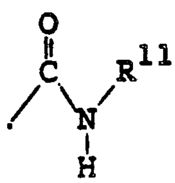 or 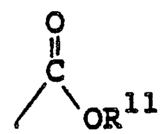,

where $R^{11}$ is hydrogen or $C_1$-$C_3$-alkyl;

x is 1 to 4;

t, u, v and w are independently 0 or 1, provided that w is not 1 when v is 0 and when t is 0, u, v, w and x are not simul taneously 0; and

Y, $R^4$, $R^{10}$ and s are as defined above;

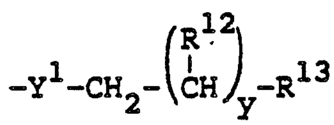,

where

$Y^1$ is -NH-, -N-$C_1$-$C_4$-alkyl- or -O-;

y is 1 to 4;

$R^{12}$ is hydrogen; hydroxy; $NH_2$; $N(C_1$-$C_4$-alkyl$)_2$; $NH(C_1$-$C_4$-alkyl); guanidyl; or $N(C_1$-$C_4$-alkyl$)\overset{\oplus}{_3}$ $A^{\ominus}$, wherein $A^{\ominus}$ is as defined above;

provided that at least one $R^{12}$ is other than hydrogen; and

$R^{13}$ is $C_1$-$C_4$-alkyl; $C_3$-$C_7$-cycloalkyl; aryl, as defined above, with the substituent(s) on the substituted aryl also alternatively selected from the group consisting of $\alpha$-aminocarboxy $C_1$-$C_4$-alkyl, $\alpha$-aminocarboxy-$C_1$-$C_4$-alkyl ester or amide, and carboxy-$C_1$-$C_4$-alkyl ester or amide; $CO_2H$; $CO_2$-$C_1$-$C_4$-alkyl; $NR^5R^6$, wherein $R^5$ and $R^6$ are defined as above; sulfo; or Het, as defined above;

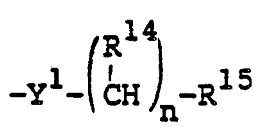,

where

$R^{14}$ is hydrogen, $CO_2H$, $CO_2$-$C_1$-$C_4$-alkyl or $NR^5R^6$, wherein $R^5$ and $R^6$ are as defined above;
$R^{15}$ is $CO_2H$; $CO_2$-$C_1$-$C_1$-$C_4$-alkyl; $NR^5R^6$, wherein $R^5$ and $R^6$ are as defined above; sulfo, or aryl, as defined above, with the substituent(s) on the substituted aryl also alternatively being selected from the group consisting of α-aminocarboxy $C_1$-$C_4$-alkyl, α-aminocarboxy $C_1$-$C_4$alkyl ester or amide, and carboxy-$C_1$-$C_4$-alkyl ester or amide, or when n is 1, $R^{15}$ is alternatively $C_1$-$C_4$-alkyl, $C_3$-$C_7$-cycloalkyl or Het, as defined above; and
$Y^1$ and n are defined as above;
except that when n is 1, neither nor $R^{15}$ is $NR^5R^6$, when n is 1 and $R^{14}$ is H, $R^{15}$ cannot be $CO_2H$ or $CO_2$-$C_1$-$C_4$-alkyl, when n is 1 and $R^{14}$ is $CO_2H$ or $CO_2$-$C_1$-$C_4$-alkyl, $R^{15}$ cannot be aryl, and when n is 2, 3, 4 or 5, the $R^{14}$ on the carbon adjacent $Y^1$ cannot be $NR^5R^6$;

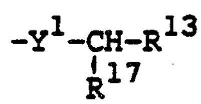 ,

where
$R^{17}$ is h or $C_1$-$C_4$-alkyl; and
$Y^1$ and $R^{13}$ are as defined above, except that $R^{13}$ in this formula cannot be $NR^5R^6$, $CO_2H$ or $CO_2$-$C_1$-$C_4$-alkyl;
or

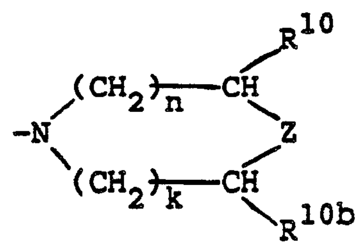

where
Z is NH, $NR^{10}$, $NR^{11a}R^{11b}$, O, S, SO $SO_2$ or $CHR^{10}$, wherein $R^{10}$ is as defined above;
k is 1-to-5; and
$R^{10b}$ is independently one of the definitions of $R^{10}$; Het, as defined above; or Het substituted by a 5- or 6-membered heterocyclic ring or benzofused heterocyclic ring, where the one or two heteroatoms are independently selected from N, O and S;
or pharmaceutically-acceptable salt thereof.

2. A peptide according to Claim 1, wherein B is Ala, Leu, Phe, HomoPhe, BisHomoPhe, Tyr, HomoTyr, Trp, or HomoTrp and E is Ala, Leu, Ser, Thr, Phe, Tyr, His, (N-methyl)His, Lys, Orn, Arg, Cys, Nle, (N-methyl)Nle, Nva, or Met.

3. A peptide according to Claim 1, which is selected from the list consisting of:
N-(2 Diethylamino)ethylcarbamoyl)Phe-His-ACHPA-NH-2(S)-methylbutyl;
N-(Methyl-2-(morpholin-4-yl)ethylcarbamoyl)Phe-His-ACHPA-NH-2(S)-methylbutyl;
N-(Methyl-3-(morpholin-4-yl)propylcarbamoyl)Phe-His-ACHPA-NH-2(S)methylbutyl;
N-(Methyl-(pyridin-4-yl)methylcarbamoyl)Phe-His-ACHPA-NH-2(S)-methylbutyl;
N-(Methyl-(p-carboxyphenyl)methylcarbamoyl)Phe-His-ACHPA-NH-2(S)-methylbutyl;
N-(Methyl-3-carboxypropylcarbamoyl)Phe-His-ACHPA-NH-2(S)-methylbutyl;
N-(Methyl-3-sulfonylpropylcarbamoyl)Phe-His-ACHPA-NH-2(S)-methylbutyl;
N-((1-(2-Butyl)-2-diethylamino)ethylcarbamoyl)Phe-His-ACHPA-NH-2(S)-methylbutyl;
N-((1-(2-Propyl)-2-diethylamino)ethylcarbamoyl)-$N^{\alpha}$-$CH_3$)Phe-His-ACHPA-NH-2(S)-methylbutyl;
N-((1-(Isobutylpyrrolidin-3-yl)carbamoyl)Phe-His-ACHPA-NH-2(S)-methylbutyl;
N-((1-(2-Hydroxy)ethylpyrrolidin-3-yl)carbamoyl)Phe-His-ACHPA-NH-2(S)-methylbutyl;
N-((1 (Carboxymethylpyrrolidin-3-yl)carbamoyl)-(α-$CH_3$)Phe-His-ACHPA-$NHCH_2CH_2N(CH_2CH_2)_2O$;
N-((1-Ethylpiperidin-4 yl)carbamoyl)Phe-His-ACHPA-NH-2(S)-methylbutyl;
N-((1-(2-Butyl)-2-(morpholin-4-yl)ethylcarbamoyl)PheHis-ACHPA-NH-2(S)-methylbutyl;
N-((1-(2-Butyl)-2-(4-methylpiperazin-1-yl))ethylcarbamoyl)Phe-His-ACHPA-NH-2(S)-methylbutyl;

N-((1-(2-Butyl)-2-(thiamorpholinesulfon-4-yl))ethylcarbamoyl)Phe-His-ACHPA-NH-2(S)-methylbutyl;
N-(2-(Morpholin-4-yl)ethylcarbamoyl)Phe-His-ACHPA-NH-2(S)-methylbutyl;
N-Methyl-2-(morpholin-4-yl)ethylcarbamoyl)-($N^{\alpha}$-$CH_3$)-Phe-His-ACHPA-NH-2(S)-methylbutyl;
N-((Morpholin-4-yl)carbonyl)Phe-His-ACHPA-NHCH(2-butyl)-CH(OH)$CH_2N(CH_2CH_2)_2O$;
N-((1-Methylpiperazin-4-yl)carbonyl)-($N^{\alpha}$-$CH_3$)Phe-His-ACHPA-NH-2(S)-methylbutyl;
N-((Thiamorpholinesulfon-4-yl)carbonyl)Phe-His-ACHPA-NHCH(2-butyl)$CH_2CH_2N(CH_2CH_2)_2O$;
N-((1-carboxymethylpiperazin-4-yl)carbonyl)-($\alpha$-$CH_3$)-Phe-His-ACHPA-NH-2(S)-methylbutyl;
N-((1-(2-Hydroxy)ethylpiperazin-4-yl)carbonyl)-$N^{\alpha}$-$CH_3$)Phe-His-ACHPA-NH-2(S)-methylbutyl;
N-((1-(2-Dimethylamino)ethylpiperazin-4-yl)carbonyl)-Phe-His-ACHPA-NH-2(S)-methylbutyl;
N-((1-(2-Dimethylcarboxamido)ethylpiperazin-4-yl)-carbonyl)Phe-His-ACHPA-NH-2(S)-methylbutyl;
N-((4-Dimethylaminopiperidin-1-yl)carbonyl)-($N^{\alpha}$-$CH_3$)-Phe-His-ACHPA-NHCH(2-butyl)$CH_2CO_2H$;
N-((3-Diethylaminopiperidin-1-yl)carbonyl)Phe-His-ACHPA-NH-2(S)-methylbutyl;
N-((2-Diethylaminomethylpyrrolidin-1-yl)carbonyl)Phe-His-ACHPA-NH-2(S)-methylbutyl;
N-((Morpholin-4-yl)carbonyl)-($N^{\alpha}$-$CH_3$)Phe-His-Cal[CH(OH)$CH_2$]Val-NH-$CH_2CH_2N(CH_2CH_2)_2O$;
N-((Morpholin-4-yl)carbonyl)Phe-His-Cal[CH(OH)$CH_2$]Val-NH-i-Bu;
N-((Morpholin-4-yl)carbonyl)Phe-($N^{\alpha}$-$CH_3$)His-Cal[CH(OH)$CH_2$]Val-NH-2(S)-methylbutyl;
N-((Morpholin-4-yl)carbonyl)Phe-His-Cal[CH(OH)$CH_2$]Val-NH$CH_2CH_2N(CH_2CH_2)_2O$;
N-(Morpholin-4-yl)carbonyl)Phe-His-Cal[CH(OH)$CH_2$]Val-NH-$CH_2$(pyridin-4-yl);
[N-((Morpholin-4-yl)carbonyl)-($N^{\alpha}$-$CH_3$)Phe-His-Cal[CH(OH)$CH_2$]Val-NH-(N-methyl-3-quinuclidinonium)-]$^{\oplus}$OAc$^{\ominus}$;
N-((Morpholin-4-yl)carbonyl)Phe-($N^{\alpha}$-$CH_3$)His-Cal[CH(OH)$CH_2$]Val-NH-2(S)-methylbutyl;
N-((Morpholin-4-yl)carbonyl)Phe-His-Cal[CH(OH)$CH_2$]Val-NH-(3-aminoquinuclidinyl);
N-((Morpholin-4-yl)carbonyl)Phe-His-Cal[CH(OH)$CH_2$]Val-NH-(N-methyl-3-aminoquinuclidinyl);
N-((Morpholin-4-yl)carbonyl)Phe-His-ACHPA-NH-(3-aminoquinuclidinyl);
[N-((Morpholin-4-yl)carbonyl)Phe-His-ACHPA-NH-(N-methyl-3-aminoquinuclidinyl)]$^{\oplus}$OAc$^{\ominus}$;
N-((4-Methylpiperazin-1-yl)carbonyl)Phe-His-ACHPA-NH-CH(2-butyl)$CH_2N(CH_3)_2$;
[N-((1-Methylpiperazin-4-yl)carbonyl)Phe-His-ACHPA-NH-CH(2-butyl)$CH_2N(CH_3)_3$]$_{\oplus}$OAc$_{\ominus}$;
N-((1-(2-Hydroxy)ethylpiperazin-4-yl)carbonyl)Phe-His-ACHPA-NH-CH(2-butyl)$CH_2N(CH_2CH_2)_2O$;
N-((4-Methylpiperazin-1-yl)carbonyl)Phe-His-ACHPA-NH-CH(2-butyl)$CH_2$NH(4,5-dihydroimidazol-2-yl);
[N-((Morpholin-4-yl)carbonyl)Phe-His-ACHPA-NH-CH(2-butyl)$CH_2N(CH_3)(CH_2CH_2)_2O$]$^{\oplus}$OAc$^{\ominus}$;
N-(2-Diethylamino)ethylsulfamoyl)Phe-His-ACHPA-NH-2(S)-methylbutyl;
N-(Methyl-(2-diethylamino)ethylsulfamoyl)-($N^{\alpha}$-$CH_3$)-Phe-His-ACHPA-NH-2(S)-methylbutyl;
N-((1-(2-Butyl)-2-diethylamino)ethylsulfamoyl)Phe-His-ACHPA-NH$(CH_2)_3N(CH_2CH_2)_2O$;
N-((1-(2-Propyl)-2-diethylamino)ethylsulfamoyl)Phe-His-ACHPA-NH$(CH_2)_3N(CH_2)_2O$;
N-((1-Isobutylpyrrolidin-3-yl)sulfamoyl)Phe-His-ACHPA-NH-2(S)-methylbutyl;
N-((1-(2-Hydroxy)ethylpyrrolidin-3-yl)sulfamoyl)Phe-His-ACHPA-NH-2(S)-methylbutyl;
N-((Morpholin-4-yl)sulfonyl)-($N^{\alpha}$-$CH_3$)Phe-His-Cal[CH(OH)$CH_2$]Val-NH-i-Bu;
N-((Morpholin-4-yl)sulfonyl)Phe-($N^{\alpha}$-$CH_3$)His-Cal[CH(OH)$CH_2$]Val-NH-2(S)-methylbutyl;
N-((Morpholin-4-yl)sulfonyl)Phe-His-Cal[CH(OH)$CH_2$]Val-NH$CH_2CH_2N(CH_2CH_2)_2O$;
N-((Morpholin-4-yl)sulfonyl)Phe-($N^{\alpha}$-$CH_3$)His-Cal[CH(OH)$CH_2$]Val-NH-$CH_2$(pyridin-4-yl);
N-([(Morpholin-4-yl)sulfonyl)-($N^{\alpha}$-$CH_3$)Phe-His-Cal[CH(OH)$CH_2$]Val-NH-(N-methyl-3-quinuclidinonium)-]$^{\oplus}$OAc$^{\ominus}$;
N-((Morpholin-4-yl)sulfonyl)Phe-($N^{\alpha}$-$CH_3$)His-Cal-[CH(OH)$CH_2$]Val-NH-2(S)-methylbutyl;
N-(Methyl-3-(morpholin-4-yl)propylsulfamoyl)-($N^{\alpha}$-$CH_3$)Phe-His-ACHPA-NH-2(S)-methylbutyl;
N-Methyl-(pyridin-4-yl)methylsulfamoyl)Phe-His-ACHPA-NHCH(2-butyl)$CH_2N(CH_2CH_2)SO_2$;
N-(Methyl-(p-carboxyphenyl)methylsulfamoyl)-($N^{\alpha}$-$CH_3$)Phe-His-ACHPA-NH-2(S)-methylbutyl; and
N-(Methyl-3-carboxypropylsulfamoyl)Phe-His-ACHPA-NH-2(S)-methylbutyl.

4. A pharmaceutical composition for renin-associated hypertension or congestive heart failure comprising a pharmaceutical carrier and a therapeutically-effective amount of a peptide according to Claim 1.

5. A pharmaceutical composition according to Claim 4, also comprising an adjuvant.

6. A pharmaceutical composition according to Claim 4, also comprising one or more compounds selected from the group consisting of:

Diuretics:

acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic

mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents:

dibenamine; phentolamine; phenoxybenzamine; prazosin; tolazoline;

β-Adrenergic Blocking Agents:

atenolol; metoprolol; nadolol; propranolol; timolol;
((±)-2-[3-(tert-butylamino)-2-hydroxypropoxy]-2-furananilide) (ancarolol);
(2-acetyl-7-(2-hydroxy-3-isopropylaminopropoxy)benzofuran HCl) (befunolol);
((±)-1-(isopropylamino)-3-(p-(2-cyclopropylmethoxyethyl)-phenoxy)-2-propranol HCl) (betaxolol);
(1-[(3,4-dimethoxyphenethyl)amino]-3-(m-tolyloxy)-2-propanol HCl) (bevantolol);
((±)-1-(4-((2-isopropoxyethoxy)methyl)phenoxy)-3-isopropylamino-2-propanol)fumarate) (bisoprolol);
(4-(2-hydroxy-3-[4-(phenoxymethyl)-piperidino]propoxy)-indole);
(carbazolyl-4-oxy-5,2-(2-methoxyphenoxy)-ethylamino-2-propanol);
(1-((1,1-dimethylethyl)amino)-3-((2-methyl-1H-indol-4-yl)oxy)-2-propanol benzoate) (bopindolol);
(1-(2-exobicyclo[2.2.1]-hept-2-ylphenoxy)-3-[(1-methylethyl)-amino]-2-propanol-HCl) bornaprolol);
(o-[2-hydroxy-3-[(2-indol-3-yl-1,1-dimethylethyl)amino]propoxy]benzonitrile HCl) (bucindolol);
(α-[(tert.butylamino)methyl]-7-ethyl-2-benzofuranmethanol) (bufuralol);
(3-[3-acetyl-4-[3-(tert.butylamino)-2-hydroxypropyl]phenyl]-1,1-diethylurea HCl) (celiprolol);
((±)-2-[2-[3-[(1,1-dimethylethyl)amino]-2-hydroxypropoxy]phenoxy]-N-methylacetamide HCl) (cetamolol);
(2-benzimidazolyl-phenyl(2-isopropylaminopropanol));
((±)-3′-acetyl-4′-(2-hydroxy-3-isopropylaminopropoxy)acetanilide HCl) (diacetolol);
(methyl-4-[2 hydroxy-3-[(1-methylethyl)aminopropoxy]]benzenepropanoate HCl) (esmolol);
(erythro-DL-1-(7-methylindan-4-yloxy)-3-isopropylaminobutan-2-ol);
(1-(tert.butylamino)-3-[O-(2-propynyloxy)phenoxy]-2-propanol (pargolol);
(1-(tert.butylamino)-3-[o-(6-hydrazino-3-pyridazinyl)phenoxy]-2-propanol diHCl) (prizidilol);
((-)-2-hydroxy-5-[(R)-1-hydroxy-2-[(R)-(1-methyl-3-phenylpropyl)amino]ethyl]benzamide);
(4-hydroxy-9-[2-hydroxy-3-(isopropylamino)-propoxy]-7-methyl-5H-furo[3,2-g][1]-benzopyran-5-one) (iprocrolol);
((-)-5-(tert.butylamino)-2-hydroxypropyl]-3,4-dihydro-1-2H)-naphthalenone HCl) (levobunolol);
(4-(2-hydroxy-3-isopropylamino-propoxy)-1,2-benzisothiazole HCl);
(4-[3-(tert.butylamino)-2-hydroxypropoxy]-N-methylisocarbostyril HCl);
((±)-N-2-[4-(2-hydroxy-3-isopropyl aminopropoxy)phenyl]ethyl-N′-isopropylurea) (pafenolol);
(3-[[(2-trifluoroacetamido)ethyl]amino]-1-phenoxypropan-2-ol);
(N-(3-(o-chlorophenoxy)-2-hydroxypropyl)-N′-(4′-chloro2,3-dihydro-3-oxo-5-pyridazinyl)ethylenediamine);
((±)-N-[3-acetyl-4-[2-hydroxy-3-[(1-methylethyl)amino]propoxy]phenyl]butanamide) (acebutolol);
((±)-4′-[3-(tert-butylamino)-2-hydroxypropoxy]spiro[cyclohexane-1,2′-indan]-1′-one) (spirendolol);
(7-[3-[[2-hydroxy-3-[(2 methylindol-4-yl)oxy]propyl]amino]butyl]thiophylline) (teoprolol);
((±)-1-tert.butylamino-3-(thiochroman-8-yloxy)-2-propanol) (tertatolol);
((±)-1-tert.butylamino-3-(2,3-xylyloxy)-2-propanol HCl) (xibenolol);
(8-[3-(tert.butylamino)-2-hydroxypropoxy]-5-methylcoumarin) (bucumolol);
(2-(3-(tert.butylamino)-2-hydroxy-propoxy)benzonitrile HCl)-(bunitrolol);
((±)-2′-[3-(tert-butylamino)-2-hydroxypropoxy-5′-fluorobutyrophenone) (butofilolol);
(1-(carbazol-4-yloxy)-3-(isopropylamino)-2-propanol) (carazolol);
(5-(3-tert.butylamino-2-hydroxy)propoxy-3,4-dihydrocarbostyril HCl) (carteolol);
(1-(tert.butylamino)-3-(2,5-dichlorophenoxy)-2-propanol) (cloranolol);
(1-(inden-4(or 7)-yloxy)-3-(isopropylamino)-2-propanol HCl) (indenolol);
(1-isopropylamino-3-[(2-methylindol-4-yl)oxy]-2-propanol) (mepindolol);
(1-(4-acetoxy-2,3,5-trimethylphenoxy)-3-isopropylaminopropan-2-ol)-(metipranolol);
(1-(isopropylamino)-3-(o-methoxyphenoxy)-3-[(1-methylethyl)amino]-2-propanol) (moprolol);
((1-tert.butylamino)-3-[(5,6,7,8-tetrahydro-cis-6,7-dihydroxy-1-naphthyl)oxy]-2-propanol)-(nadolol);
((S)-1-(2-cyclopentylphenoxy)-3-[(1,1-dimethylethyl)amino]-2-propanol sulfate (2:1)) (penbutolol);

(4′-[1-hydroxy-2-(amino)ethyl]methanesulfonanilide) (sotalol);
(2-methyl-3-[4-(2-hydroxy-3-tert.butylaminopropoxy)phenyl]-7-methoxy-isoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxypropoxy]-β-methylcinnamonitrile) (pacrinolol);
((±)-2-(3′-tert.butylamino-2′-hydroxypropylthio)-4-(5′-carbamoyl-2′-thienyl)thiazole HCl) (arotinolol);
((±)-1-[p-[2-(cyclopropylmethoxy)ethoxy]phenoxy]-3-(isopropylamino)-2-propanol) (cicloprolol);
((±)-1-[(3-chloro-2-methylindol-4-yl)oxy]-3-[(2-phenoxyethyl)amino]-2-propanol) (indopanolol);
((±)-6-[[2-[[3-(p-butoxyphenoxy)-2-hydroxypropyl]amino]ethyl]amino]-1,3-dimethyluracil) (pirepolol);
(4-(cyclohexylamino)-1-(1-naphtholenyloxy)-2-butanol);
(1-phenyl-3-[2-[3-(2-cyanophenoxy)-2-hydroxypropyl]aminoethyl]hydantoin HCl);
(3,4-dihydro-8-(2-hydroxy-3-isopropylaminopropoxy)-3-nitroxy-2H-1-benzopyran) (nipradolol);

α- and β-Adrenergic Blocking Agents:

((±)-1-tert-butylamino)-3-[o-[2-(3-methyl-5-isoxazolyl)vinyl]phenoxy]-2-propanol) (isoxaprolol);
(1-isopropylamino-3-(4-(2-nitroxyethoxy)phenoxy)-2-propanol HCl);
(4-hydroxy-α-[[3-(4-methoxyphenyl)-1-methylpropyl]aminomethyl]-3-(methylsulfinyl)-benzmethanol HCl) (sulfinalol);
(5-[1-hydroxy-2-[[2-(o-methoxyphenoxy)ethyl]amino]ethyl]-2-methylbenzenesulfonamide HCl);
(5-[1-hydroxy-2-[(1-methyl-3-phenylpropyl)amino]ethyl]salicylamide HCl) (labetalol);
(1-((3-chloro-2-methyl-1H-indol-4-yl)oxy)-3-((2-phenoxyethyl)amino)-2-propanol-hydrogenmalonate) (ifendolol);
(4-(2-hydroxy-3-[(1-methyl-3-phenylpropyl)amino]propoxy)benzeneacetamide);
(1-[3-[[3-(1-naphthoxy)-2-hydroxypropyl]-amino]-3,3-dimethyl-propyl]-2-benzimidazolinone);
(3-(1-(2-hydroxy-2-(4-chlorophenylethyl)-4-piperidyl)-3,4-dihydroxy)quinoxolin-2(1H)-one);

CNS-Acting Agents:

clonidine; methyldopa;

Adrenergic Neuron Blocking Agents:

guanethidine; reserpine and other rauwolfia alkaloids such as rescinnamine;

Vasodilators:

diazoxide; hydralazine; minoxidil;

Angiotensin I Converting Enzyme Inhibitors:

1-(3-mercapto-2-methyl-1-oxopropyl)-L-proline (captopril);
(1-(4-ethoxycarbonyl-2,4(R,R)-dimethylbutanoyl)indoline-2(S)-carboxylic acid);
(2-[2-[[1-(ethoxycarbonyl)-3-phenyl-propyl]amino]-1-oxopropyl]-1,2,3,4-tetrahydro-3-isoquinoline carboxylic acid);
((S)-1-[2-[[1-(ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)-cis,syn-octahydroindol-2(S)-carboxylic acid HCl);
((-)-(S)-1-[(S)-3-mercapto-2-methyl-1-oxopropyl]indoline-2-carboxylic acid);
[1(S),4S]-1-[3-(benzoylthio)-2-methyl-1-oxopropyl]-4-phenylthio-L-proline;
(3-([1-ethoxycarbonyl-3-phenyl-(1S)-propyl]amino)-2,3,4,5-tetrahydro-2-oxo-1-(3S)-benzazepine-1-acetic acid HCl);

(N-(2-benzyl-3-mercaptopropanoyl)-S-ethyl-L-cysteine) and the S-methyl analogue;
(N-((1(S)-ethoxycarbonyl-3-phenylpropyl)-L-alanyl-L-proline maleate) (enalapril);
N-[1-(S)-carboxy-3-phenylpropyl]-L-alanyl-1-proline;
$N^2$-[1-(S)-carboxy-3-phenylpropyl]-L-lysyl-L-proline (lisinopril);

Calcium Channel Blockers:

α-[3-[[2-(3,4-dimethoxyphenyl)ethyl]methylamino]propyl]-3,4-dimethoxy-α-(1-methylethyl)benzeneacetonitrile (verapamil);
1,4-dihydro-2,6-dimethyl-4-(2-nitrophenyl)-3,5-pyridinedicarboxylic acid dimethyl ester (nifedipine);
2-(2,2-dicyclohexylethyl)piperidine (perhexiline);
N-(1-methyl-2-phenylethyl)- -phenylbenzenepropanamine (prenylamine);
3-(aminosulfonyl)-4-chloro-N-(2,3-dihydro-2-methyl-1H-indol-1-yl)benzamide (indapamide);
(2′-(2-diethylaminoethoxy-3-phenylpropiophenone (etafenone);
(4-[4,4-bis-(4-fluorophenyl)butyl]-N-(2,6-dimethylphenyl)-1-piperazineacetamide) (lidoflazine);
(2-(N-benzyl-N-methylamino)ethylmethyl-2,6-dimethyl-4-(m-nitrophenyl)-1,4-dihydro-3,5-pyridinedicarboxylate HCl) (nicardipine);
(N-(3,4-dimethoxyphenethyl)-2-(3,4-dimethoxyphenyl)-N-methyl-m-dithiane-2-propylamine-1,1,3,3-tetraoxide) (tiapamil);
(5,6-dimethoxy-2-(3-[(α-(3,4-dimethoxy)phenylethyl)methylamino]propyl)phthalimidine) (falipamil);
(β-[(2-methylpropoxy)methyl]-N-phenyl-N-phenylmethyl-1-pyrrolidineethanamine HCl monohydrate) (bepridil);
((+)-cis-3-(acetyloxy)-5-[2-(dimethylamino)ethyl]-2,3-dihydro-2-(4-methoxyphenyl)-1,5-benzothiazepin-4-(5H)-one) (diltiazem);
((E)-1-[bis-(p-fluorophenyl)methyl]-4-cinnamylpiperazine di HCl) (flunarizine);
(5-[(3,4-dimethoxyphenethyl)methylamino]-2-isopropyl-2-(3,4,5-trimethoxyphenyl)valeronitrile (gallopamil);
(ethylmethyl(2,3-dichlorophenyl)-1,4-dihydro-2,6-dimethyl-3,5-pyridinedicarboxylate (felodipine);
(isopropyl-2-methoxyethyl-1,4-dihydro-2,6-dimethyl-4-(3-nitrophenyl)-3,5-pyridinecarboxylate) (nimodipine);
(3-ethyl-5-methyl-1,4-dihydro-2,6-dimethyl-4-(3-nitrophenyl)-3,5-pyridine-dicarboxylate) (nitrendipine); and

Other Artihypertensive Agents:

aminophylline; cryptenamine acetates and tannates; deserpidine; meremethoxylline procaine; pargyline; trimethaphan camsylate; and the like, as well as admixtures and combinations thereof.

7. The use of a peptide as claimed in Claim 1 for the preparation of a medicament useful for treating renin-associated hypertension or congestive heart failure in mammals.

8. The use as claimed in Claim 7 wherein mammals are human and the amount administered is from 0.02 to 10 grams per day.